(19)

Europäisches
Patentamt

European
Patent Office

Office européen
des brevets

(11) **EP 4 220 657 A1**

(12) **EUROPEAN PATENT APPLICATION**

(43) Date of publication:
**02.08.2023 Bulletin 2023/31**

(21) Application number: **22206609.4**

(22) Date of filing: **10.11.2022**

(51) International Patent Classification (IPC):
**G16H 40/00** (2018.01)     **G06Q 10/0631** (2023.01)

(52) Cooperative Patent Classification (CPC):
**G16H 40/00; G06Q 10/0631; G06Q 50/22;
G06Q 50/26**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.01.2022 JP 2022013730**

(71) Applicant: **FUJITSU LIMITED
Kawasaki-shi, Kanagawa 211-8588 (JP)**

(72) Inventors:
• **Sasamoto, Yuki
Kawasaki-shi, Kanagawa, 211-8588 (JP)**

• **Inomata, Akihiro
Kawasaki-shi, Kanagawa, 211-8588 (JP)**
• **Hotta, Shinji
Kawasaki-shi, Kanagawa, 211-8588 (JP)**
• **Amemiya, Satoshi
Kawasaki-shi, Kanagawa, 211-8588 (JP)**
• **Katsura, Daichi
Kawasaki-shi, Kanagawa, 211-8588 (JP)**

(74) Representative: **Hoffmann Eitle
Patent- und Rechtsanwälte PartmbB
Arabellastraße 30
81925 München (DE)**

(54) **PLANNING METHOD AND PLANNING PROGRAM**

(57)     A computer-implemented method of performing planning processing, the method including: acquiring information of a menu of a predetermined service and information of a stakeholder related to the service; calculating a change frequency of execution of the menu for a combination of the stakeholder and the menu; specifying a menu to be recommended on the basis of the calculated change frequency; and outputting the specified menu to the stakeholder of the calculated combination.

FIG. 1

EP 4 220 657 A1

**Description**

FIELD

The embodiments discussed herein are related to a planning method and a planning program.

BACKGROUND

In recent years, toward implementation of digital transformation (DX), there have been high expectations for collecting various types of information in existing operations as data, and improving the existing operations and promoting efficiency of the existing operations using the collected data. For example, in various industries, there is a technique for recommending a specific menu on the basis of expectations of all stakeholders involved in a service to be provided as support for planning and implementation of the service. The stakeholders include, for example, a planner who plans a service, a provider who provides the service, and a user who receives the service.

Traditionally, services such as examinations, treatments, and rehabilitation (rehabilitation) plans in administration, hospitals, nursing care facilities, and the like have been manually performed. However, advances in AI and machine learning techniques have made it possible to automatically plan and implement services, and data-driven techniques for recommending menus of effective services have been developed.

As a prior art related to menu recommendation of planning, for example, there is a technique for recommending a personal best menu that is expected to maximize effects such as improvement of a care level for a target user on the basis of similar cases in the past. Furthermore, for example, there is a technique for prioritizing and recommending a menu of rehabilitation plans in consideration of user's own interest and risk of illness or the like by profiling the user. Furthermore, for example, there is a technique for recommending a rehabilitation menu on the basis of user's prior evaluation, taking into account the user's difficulty level in implementation and effects. Furthermore, for example, there is a technique for determining a correlation between a positive outcome from patient outcome data and medical product location data and generating a medical suggestion for changing a medical resource use practice on the basis of the correlation. Furthermore, for example, there is a technique for associating each piece of information of a nursing care plan, nursing achievement, an evaluation level, a creator of the nursing care plan, and a medical staff in charge to one another each other when the nursing care plan is changed, storing an individual nursing care plan information with the number of times of changes in a database, and extracting the individual nursing care plan information according to a condition specified by the medical staff. Furthermore, for example, there is a technique for obtaining a probability of improvement in a level of care needed in a care plan in which a care recipient is taken care of, changing a model of the care plan, and outputting the care plan that can be expected to improve the level of care needed. Furthermore, for example, there is a technique for updating a guidance on the basis of a risk of re-hospitalization and dissatisfaction of a patient, and costs, and optimizing achievement of a care plan goal through model learning. Furthermore, for example, there is a technique for selecting a patient nursing care plan and a monitoring action according to a patient care plan on the basis of individual patient actions and lifestyle, and changing a monitoring plan as the patient care plan changes over time.

Examples of the related art include: Japanese Laid-open Patent Publication No. 2020-166835; Japanese National Publication of International Patent Application No. 2021-509505; Japanese Laid-open Patent Publication No. 2008-165358; International Publication Pamphlet No. WO 2018/030340; U.S. Patent No. 10923233; U.S. Patent Application Publication No. 2017/0300637; Tomomi Ogawa, Hiroki Matsumoto, "Improvement of Rehabilitation Menu Recommendation System with Extended Nursing Care Need Level Determination Period", Journal of Telemedicine Society of Japan, 16(2), 152-155, 2020; and "It is possible to propose an optimal rehabilitation plan that matches the characteristics of each user of nursing care services ~ Verifi cation of the eff ectiveness of individual optimal technology using digital data in nursing rehabilitation ~", [Retrieved on January 16, 2022], Internet <URL: https://www.nttdata-strategy.com/newsrelease/210701.html>, NTT DATA INSTITUTE OF MANAGEMENT CONSULTING, INC, and others, July 1, 2021.

SUMMARY

TECHNICAL PROBLEM

However, in an existing technique, for example, when a plan change occurs due to sudden cancellation, change of service or the like, a recommended menu is not implemented, and an initially expected effect at the time of recommendation has not been able to be obtained. Since the existing technique does not take into consideration convenience and circumstances of each of the planner, provider, and user, it does not take into consideration the occurrence of a plan change in implementing the planned menu. In a case where the recommended menu is not implemented, the effects expected at the time of planning are not able to be obtained. In the existing technique, menu recommendation including actual circumstances of the service implementation has not been performed.

In one aspect, an object of the present embodiment is to enable recommendation of a menu of services that are highly likely to be implemented.

SOLUTION TO PROBLEM

According to an aspect of the embodiments, there is provided a computer-implemented method of performing planning processing, the method includes: acquiring information of a menu of a predetermined service and information of a stakeholder related to the service; calculating a change frequency of execution of the menu for a combination of the stakeholder and the menu; specifying a menu to be recommended on the basis of the calculated change frequency; and outputting the specified menu to the stakeholder of the calculated combination.

ADVANTAGEOUS EFFECTS OF INVENTION

According to one aspect, it becomes possible to recommend a menu of a service that is highly likely to be implemented.

BRIEF DESCRIPTION OF DRAWINGS

FIG. 1 is an explanatory diagram illustrating an example of a planning method according to an embodiment;
FIG. 2 is a diagram illustrating a configuration example of a planning system;
FIG. 3 is a block diagram illustrating a hardware configuration example of a planning device;
FIG. 4 is a block diagram illustrating a functional configuration example of the planning device;
FIG. 5A is a chart illustrating a data example in a DB retained by a client (part 1);
FIG. 5B is a chart illustrating a data example in a DB retained by a client (part 2);
FIG. 5C is a chart illustrating a data example in a DB retained by a client (part 3);
FIG. 6A is a chart illustrating a data example in a DB retained by the planning device (part 1);
FIG. 6B is a chart illustrating a data example in a DB retained by the planning device (part 2);
FIG. 6C is a chart illustrating a data example in a DB retained by the planning device (part 3);
FIG. 6D is a chart illustrating a data example in a DB retained by the planning device (part 4);
FIG. 7 (i.e., FIGs. 7A to 7D) is an explanatory diagram of an outline of processing performed by the planning device;
FIGs. 8A to 8C are explanatory diagrams of processing of estimating response change risks;
FIG. 9 is an explanatory diagram of extraction processing of conditions that minimize the response change risks;
FIGs. 10A and 10B are explanatory diagrams of expected value calculation processing in consideration of a workflow;
FIG. 11 is a flowchart illustrating an overall processing example of the planning device;
FIG. 12 is a flowchart illustrating a calculation processing example of an expected value and a response change risk in consideration of a workflow;
FIG. 13 is a flowchart illustrating a processing example of extraction of a response change risk minimum condition;
FIG. 14 is a flowchart illustrating a processing example of scheduling of a response change risk and an expected value;
FIG. 15A is explanatory diagrams illustrating an example of a user and a provider of a service;
FIG. 15B is a diagram illustrating an example of plan data and achievement data;
FIG. 16A is an explanatory diagram of an estimation example of a response change risk (part 1);
FIG. 16B is an explanatory diagram of an estimation example of a response change risk (part 2);
FIG. 17 is a diagram illustrating an estimation example of expected values for each stakeholder;
FIG. 18A is an explanatory diagram of a combination example that minimizes a response change risk;
FIG. 18B is an explanatory diagram of a combination example that cannot minimize a response change risk;
FIG. 19 (FIGs. 19A and 19B) is an explanatory diagram of similarity of estimated expected values;
FIG. 20A is a diagram illustrating a display example of a recommended menu (part 1); and
FIG. 20B is a diagram illustrating a display example of a recommended menu (part 2).

DESCRIPTION OF EMBODIMENTS

Hereinafter, embodiments of a planning method and a planning program will be described in detail with reference to the drawings.

(Example of Planning Method According to Embodiment)

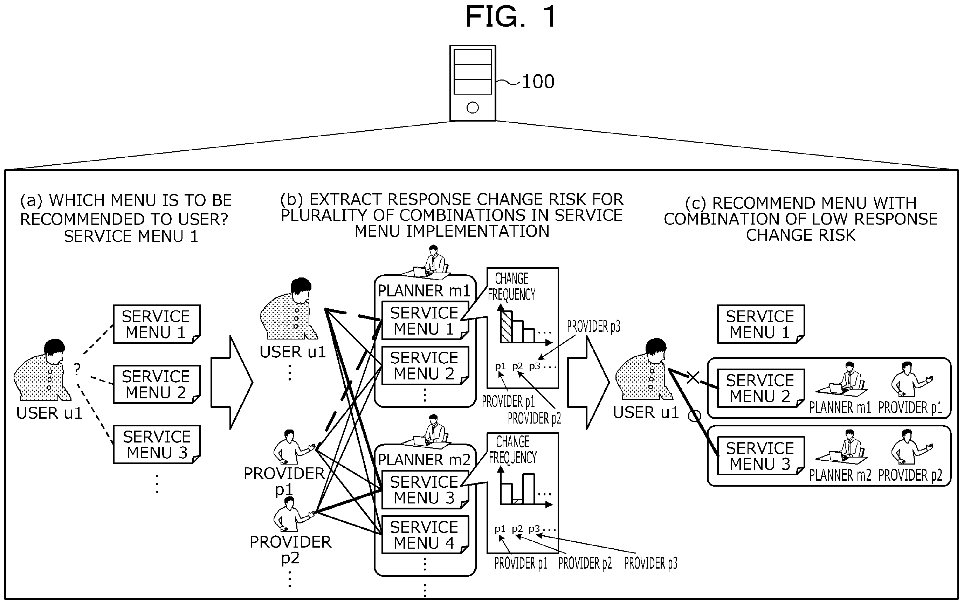

FIG. 1 is an explanatory diagram illustrating an example of a planning method according to an embodiment. A planning device 100 acquires information of a menu of a predetermined service and information of stakeholders related to the service. The planning device 100 calculates a change frequency of menu execution for combinations of stakeholders and menus. The planning device 100 specifies a menu to be recommended on the basis of the calculated change frequency. Then, the planning device 100 outputs the specified menu to the stakeholders of the calculated combination. The planning device 100 is a computer for performing these pieces of processing.

The planning device 100 is, for example, a server or a personal computer (PC), and includes a control unit that controls the planning device 100. The planning device 100 may be a cloud server, and functions of the control unit may be arbitrarily arranged on a cloud.

In the description of the embodiment, among the stakeholders, a planner plans a certain service, for example, a rehabilitation plan menu. The provider outputs the rehabilitation plan menu planned by the planner to the user. The user performs rehabilitation based on the rehabilitation plan menu provided by the provider.

A "plan change" to be described in the embodiment is a change in implementing the planned service menu, and the plan change occurs according to arbitrariness/intention of the stakeholders who are parties concerned. There are following examples of the plan change.

A certain user "wrongly got a time" and didn't get/took a long time to get a menu i.

A certain user stopped receiving the menu i "because of previous failures".

A certain provider "took a long time to prepare" and could not/took a long time to provide the menu i.

A certain provider stopped providing the menu i because "special equipment broken down".

A certain planner has changed a provision location of the menu i "for a group (a joint with other users)". In this case, the user will not be able to receive the menu i at the original provision location. To receive the menu i, for example, the user needs to move to the provision location after the change.

A certain planner has changed a provision time of the menu i "in response to a change in a regulation (insurance coverage)". In this case, the user will not be able to receive the menu i at the initial provision time. To receive the menu i, for example, the user needs to visit the provision location again at the changed provision time.

Furthermore, a "planned menu" means that "a certain provider provides a menu of a certain service to a certain user (group) at certain timing (date and time) at a certain location (facility), which is planned by a certain planner."

As described above, the problem is that a plan change occurs in the service menu. For example, the plan change indicates that the service menu is changed in terms of time or content. Such a plan change occurs due to a response change of the stakeholder to the service menu. The response change corresponds to, for example, achievement of an actual situation in which the user does not (cannot) implement the service for the plan to implement the service. When any one of the stakeholders of the planner, the provider, and the user has the response change, the plan change will be performed for the menu.

According to the above-description, the inventors have focused on the fact that if the service menu can be recommended without causing the response change, it will be possible to suppress the plan change and implement the service with an initial effect. For this reason, the planning device 100 according to the embodiment focuses on the response change of a plurality of the stakeholders to the service menu, minimizes a response change risk, and enables recommendation of a menu that maximizes effects.

In the embodiment, for each stakeholder, a menu that can maximize the effect for the user who actually implements the menu is recommended. For example, for the user, a combination that minimizes the response change to the menu is recommended. Furthermore, for the provider, a schedule of each user is recommended with the response change risk as a constraint condition.

The planning device 100 according to the embodiment includes and implements the following processes 1. to 4. as a planning method.

1. Extraction of Response Change Points

The planning device 100 extracts a menu in which the response change has occurred from a difference between a service plan and past achievement. For example, the planning device 100 sequentially accumulates and stores a history of changes in past service menus in a storage unit. The planning device 100 extracts the menus in which the response change has occurred on the basis of the history of changes in menus at predetermined timing, for example, for each predetermined timing.

2. Calculation of Response Change Risk

The planning device 100 calculates the response change risk (change frequency) for each stakeholder (planner, user, or provider) for the extracted menu. For example, the planning device 100 extracts a changed service menu and an unchanged service menu from past data, and calculates the response change risk between the stakeholders from past data. Then, the planning device 100 calculates a range of a parameter for each service menu for each party concerned. For example, a group of service menus created by a planner m, a group of service menus provided by a provider p, and a group of service menus used by a user u are classified into respective data groups, and each response change risk is calculated. Note that m is an abbreviation for menu planner, p for provider, and u for user.

3. Extraction of Condition that minimizes Response Change Risk

The planning device 100 extracts, for a certain menu, a combination of service menus that minimizes the response change risk. For example, in a case where a change frequently occurs in the menu i in the service menu planned by planner m, the planning device 100 determines that a contribution to the response change risk of the planner m for the menu i is high. In this case, the planning device 100 performs processing of not recommending the service menu planned by the planner m as a service of the user u who is supposed to receive the menu i.

4. Recommendation of Menu that Maximizes Effect for User Under Extracted Condition

The planning device 100 recommends the menu that maximizes the effect for the user who will implement the menu to the user by display output or the like under the extracted condition extracted in 3. above.
A recommendation example of a menu by the planning device 100 will be described with reference to FIG. 1. As illustrated in FIG. 1(a), it is assumed that there is a plurality of menus 1 to n in services for a certain user u1. In this case, the planning device 100 determines which of the menus 1 to n to recommend to the user u1.
As illustrated in FIG. 1(b), the planning device 100 extracts the response change risks (change frequencies) for a plurality of combinations in implementation of the menus 1 to n of a certain service. For example, the user u1 can implement the menus 1 to n planned by a plurality of planners m1 to mn by provision by a plurality of providers p1 to pn.
In the example of FIG. 1(b), the planning device 100 calculates the response change risk (change frequency) for each of the providers p1 and p2 for the menus 1 and 2 planned by the planner m1 for the user u1. Regarding the menu 1, the example of FIG. 1(b) indicates that the response change risk (change frequency) of the provider p1 is the highest.
Similarly, the planning device 100 calculates the response change risk (change frequency) for each of the providers p1 and p2 for the menus 3 and 4 planned by a planner m2 for the user u1. Regarding the menu 3, the example of FIG. 1(b) indicates that the response change risk (change frequency) of the provider p2 is the lowest.
Then, the planning device 100 recommends, to the user, the menu by the combination of the stakeholders with the low response change risk (change frequency) illustrated in FIG. 1(b). In the example of FIG. 1(c), the planning device 100 recommends the menu 3 planned by the planner m2 and provided by the provider p2 to the user u1. The recommendation of menu 3 to the user u1 is based on the fact that the response change risk (change frequency) of the menu 3 in the combination of the planner m2 and the provider p2 illustrated in FIG. 1(b) is low. In this case, for example, no recommendation is made to the user u1 because the response change risk (change frequency) of the menu 1 in the combination of the planner m1 and the provider p1 is high.
In the example of FIG. 1(c), for the sake of convenience, the planning device 100 recommends one menu with the lowest response change risk (change frequency) to the user u1 by combining the stakeholders, but the present embodiment is not limited to this example. Although details will be described below, the planning device 100 may recommend a plurality of menus that minimizes the response change risk (change frequency) to the user u1. Furthermore, the planning device 100 may also present a schedule for implementing each menu. Moreover, the planning device 100 may recommend the response change risk for each menu to the user u1 as a constraint condition and makes the menu selectable in the state of causing the user u1 to recognize the response change risk.
According to the embodiment, the planning device 100 recommends, to the stakeholder, the menu that can be implemented as planned on the basis of the response change risk (change frequency) that occurs before the planned service menu is actually implemented. Furthermore, the planning device 100 can recommend the optimum menu in consideration of an expectation of each stakeholder for the menu. Furthermore, according to the planning device 100, it is possible to perform scheduling combining more accurate and feasible menus by using the response change risk as the constraint condition for menu assignment.

(Configuration Example of Planning System)

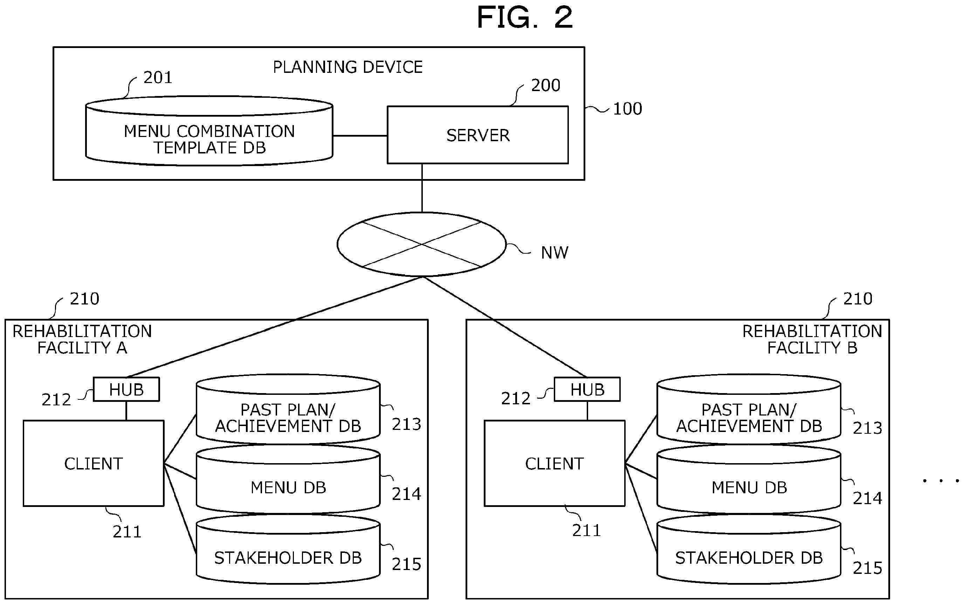

FIG. 2 is a diagram illustrating a configuration example of a planning system. In the example illustrated in FIG. 2, the planning device 100 recommends a menu for a past plan/achievement DB 213 for a rehabilitation plan of the user. The planning device 100 includes a server 200 and a menu combination template database (DB) 201. The server 200 is connected for communication with clients 211 of rehabilitation facilities A to N (210) used by the user via a network NW.

The rehabilitation facilities A to N (210) include the client 211, a hub (HUB) 212 connected for communication with the network NW, the past plan/achievement DB 213, a menu DB 214, and a stakeholder DB 215. Each of the planner, the provider, and the user as the stakeholders accesses the client 211 and input information, and the input information about each stakeholder is stored in the stakeholder DB 215. The past plan/achievement DB 213 retains plan data planned as a rehabilitation service and achievement data of a result of implementing the plan data. The menu DB 214 retains information regarding a rehabilitation menu planned by the planner.

The clients 211 of the rehabilitation facilities A to N (210) transmit the information stored in the past plan/achievement DBs 213, the menu DBs 214, and the stakeholder DBs 215 to the server 200 of planning device 100.

The server 200 of the planning device 100 performs the above-described processes 1 to 4 on the basis of the information stored in the past plan/achievement DBs 213, the menu DBs 214, and the stakeholder DBs 215 of the rehabilitation facilities A to N (210). The planning device 100 creates the menu combination template DB 201 by executing the processes 1 to 4. The menu combination template DB 201 retains information of a plurality of menu combinations related to rehabilitation. The menu combination template DB 201 may include an order of menu implementation.

The planning device 100 recommends, to the user, the menu that can be implemented as planned on the basis of content stored in the menu combination template DB 201. For example, the planning device 100 transmits and outputs information regarding the menu that can be implemented by the user to the clients 211 of the rehabilitation facilities A to N (210). The user receives the information of the menu that can be implemented on the basis of displayed content of the client 211. Note that the client 211 may be configured to transfer the information regarding the menu that can be implemented to a smart phone or the like carried by the user.

(Hardware Configuration Example of Planning Device 100)

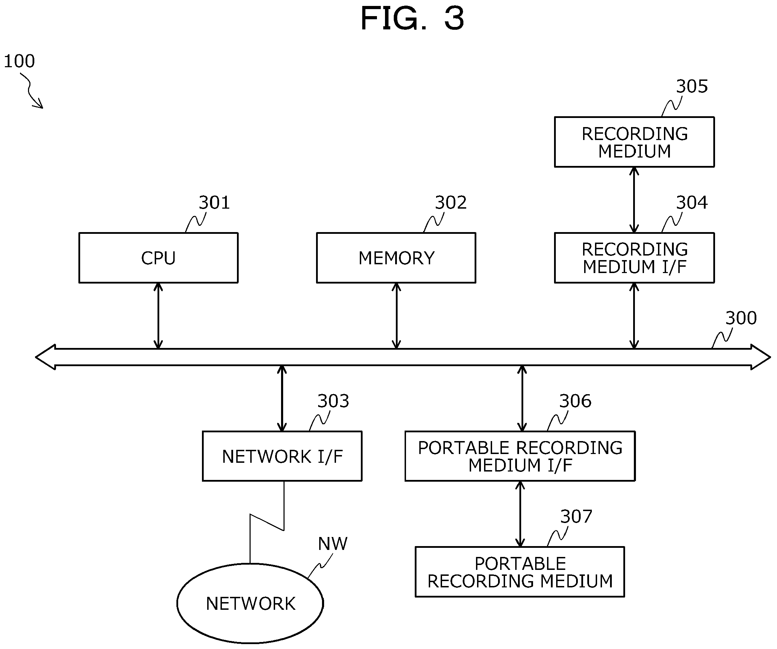

Next, a hardware configuration example of the planning device 100 will be described with reference to FIG. 3.

FIG. 3 is a diagram illustrating a hardware configuration example of the planning device. In FIG. 3, the planning device 100 includes a central processing unit (CPU) 301, a memory 302, a network interface (I/F) 303, a recording medium I/F 304, and a recording medium 305. Furthermore, a portable recording medium I/F 306 and a portable recording medium 307 are included. Furthermore, the configuration units are connected to one other by a bus 300. The network NW is, for example, a local area network (LAN), a wide area network (WAN), the Internet, or the like.

Here, the CPU 301 performs overall control of the planning device 100. The memory 302 includes, for example, a read only memory (ROM), a random access memory (RAM), a flash ROM, and the like. For example, for example, the flash ROM or the ROM stores various programs, and the RAM is used as a work area for the CPU 301. The programs stored in the memory 302 are loaded into the CPU 301 to cause the CPU 301 to execute coded processing.

The network I/F 303 is connected to the network NW through a communication line, and is connected to another computer via the network NW. Then, the network I/F 303 manages an interface between the network NW and the inside, and controls input and output of data from another computer. The network I/F 303 is, for example, a modem, a LAN adapter, or the like.

The recording medium I/F 304 controls read and write of data from and to the recording medium 305 under the control of the CPU 301. The recording medium I/F 304 is, for example, a disk drive, a solid state drive (SSD), a universal serial bus (USB) port, or the like. The recording medium 305 is a nonvolatile memory that stores data written under the control of the recording medium I/F 304. Examples of the recording medium 305 include a magnetic disk, an optical disk, and the like.

The portable recording medium I/F 306 controls read/write of data from/to the portable recording medium 307 under the control of the CPU 301. The portable recording medium 307 stores data written under the control of the portable recording medium I/F 306. Examples of the portable recording medium 307 include a compact disc (CD)-ROM, a digital versatile disk (DVD), a universal serial bus (USB) memory, and the like.

The planning device 100 may include, for example, a keyboard, a mouse, a display, a printer, a scanner, a microphone, a speaker, or the like, in addition to the above-described configuration units Furthermore, the planning device 100 may include a plurality of the recording medium I/Fs 304 and recording media 305. Furthermore, the planning device 100 does not have to include the recording medium I/F 304 or the recording medium 305.

Since a hardware configuration example of the client 211 illustrated in FIG. 2 is similar to the hardware configuration example of the planning device 100 illustrated in FIG. 3, description thereof is omitted.

(Functional Example of Planning Device)

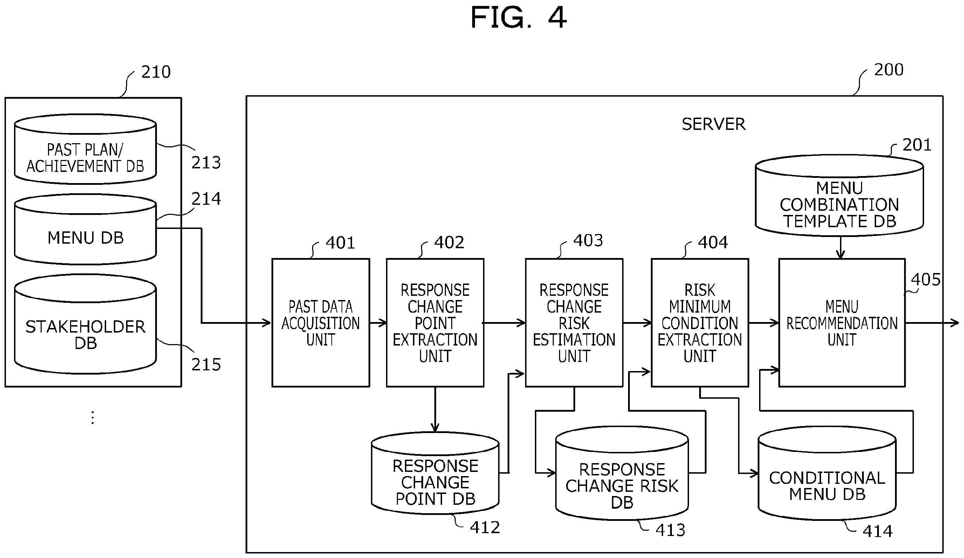

FIG. 4 is a block diagram illustrating a functional configuration example of the planning device. FIG. 4 illustrates each function of the server 200 illustrated in FIG. 2 and a plurality of DBs. The server 200 includes each of functions of a past data acquisition unit 401, a response change point extraction unit 402, a response change risk estimation unit 403, a risk minimum condition extraction unit 404, and a menu recommendation unit 405. DBs includes the above-described menu combination template DB 201, a response change point DB 412, a response change risk DB 413, and a conditional menu DB 414.

The past data acquisition unit 401 to the menu recommendation unit 405 corresponding to a control unit of the server 200 illustrated in FIG. 4 can be functionally implemented by the CPU 301 illustrated in FIG. 3 executing a program stored in the memory 302 or the like. Furthermore, the DBs 412 to 414 of the server 200 illustrated in FIG. 4 can be functionally implemented using the memory 302, the recording medium 305, or the like illustrated in FIG. 3. Furthermore, the DBs 213 to 215 on the side of the rehabilitation facility 210 (client 211) illustrated in FIG. 4 can be functionally implemented using the memory 302, the recording medium 305, or the like illustrated in FIG. 3.

The past data acquisition unit 401 accesses the client 211 of each of the rehabilitation facilities A to N (210) and acquires information stored in the past plan/achievement DB 213, the menu DB 214, and the stakeholder DB 215.

The response change point extraction unit 402 performs processing of extracting a response change point of the above-described process 1. on the basis of the information acquired by the past data acquisition unit 401. The response change point extraction unit 402 extracts information regarding the response change for each menu, and stores the extracted information of the response change in the response change point DB 412.

The response change risk estimation unit 403 performs estimation of the response change risk of the above-described process 2. on the basis of the information of the response change point extracted by the response change point extraction unit 402. The response change risk estimation unit 403 estimates the response change risk of each menu by referring to the response change point DB 412, and stores information of the estimated response change risk in the response change risk DB 413.

The risk minimum condition extraction unit 404 extracts a condition that minimizes the response change risk of the above-described process 3. on the basis of the information of the response change risk estimated by the response change risk estimation unit 403. The risk minimum condition extraction unit 404 extracts the condition that minimizes the response change risk for each menu, for example, information of a combination of the stakeholders, by referring to the response change risk DB 413. Then, the risk minimum condition extraction unit 404 stores the menu with the extracted condition that minimizes the response change risk in the conditional menu DB 414.

The menu recommendation unit 405 performs recommendation of the menu that maximizes the effect for the user under the condition extracted in the above-described process 4. The menu recommendation unit 405 determines a menu to recommend, including information of the order in which a plurality of menus is combined, by referring to the menu combination template DB 201. Furthermore, the menu recommendation unit 405 recommends the information of the condition (such as the combination of stakeholders for each menu) that minimizes the response change risk to the stakeholders (user and provider) by referring to the conditional menu DB 414.

(Data Example of Each Database)

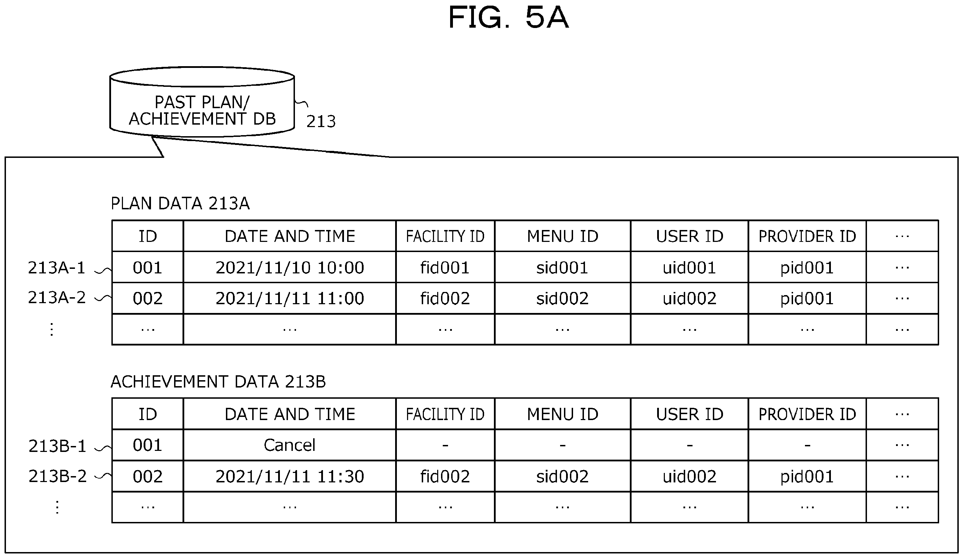

FIGs. 5A to 5C are charts illustrating data examples in a DB retained by a client. A data example in the DBs 213 to 215 of each of the rehabilitation facilities A to N illustrated in FIG. 2 will be described.

FIG. 5A illustrates the past plan/achievement DB 213. The past plan/achievement DB 213 includes plan data 213A planned by the planner and achievement data 213B corresponding to the plan data 213A.

The plan data 213A is stored as a record 213A-a in which information is set in each field on the basis of information planned by the planner. a is an arbitrary integer. For example, each field stores information such as an identifier (ID) for each record, date and time, a facility ID, a menu ID, a user ID, and the like. For example, the record 213A-1 has the ID "001", the date and time "2021/11/10 10:00", the facility ID "fid001", the menu ID "sid001", the user ID "uid001", and the provider ID "pid001".

The achievement data 213B is stored as a record 213B-a in which information is set in each field as achievement for the plan data 213A planned by the planner. For example, each field stores information such as an ID for each record, date and time, a facility ID, a menu ID, a user ID, and the like, similarly to the plan data 213A. Here, a record 213B-1 indicates that the menu ID "sid001" of the ID "001" of the record 213A-1 of the plan data 213A has not been implemented, and in this case, "Cancel" is set in the date and time.

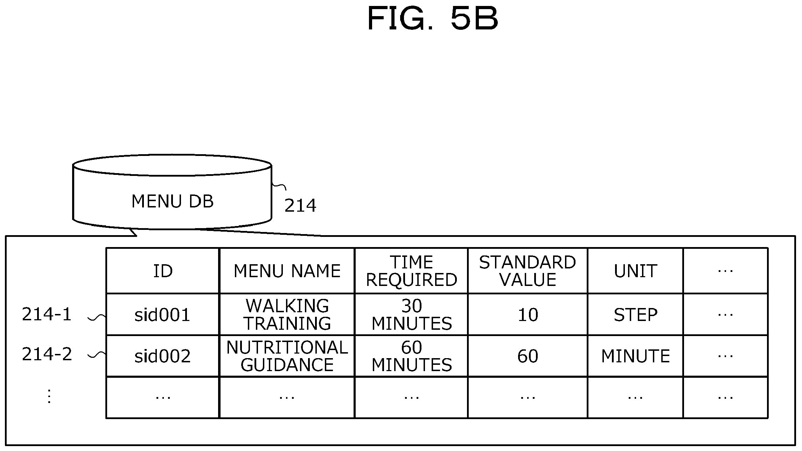

FIG. 5B illustrates the menu DB 214. The menu DB 214 is stored as a record 214-a in which information is set in each field on the basis of the information related to the rehabilitation menu planned by the planner. For example, each field stores information such as a menu ID, a menu name, a time required, a standard value, a unit, and the like. For example, the record 214-1 stores information such as the menu ID "sid001", the menu name "walking training", the time required "30 minutes", the standard value "10", the unit "step", and the like.

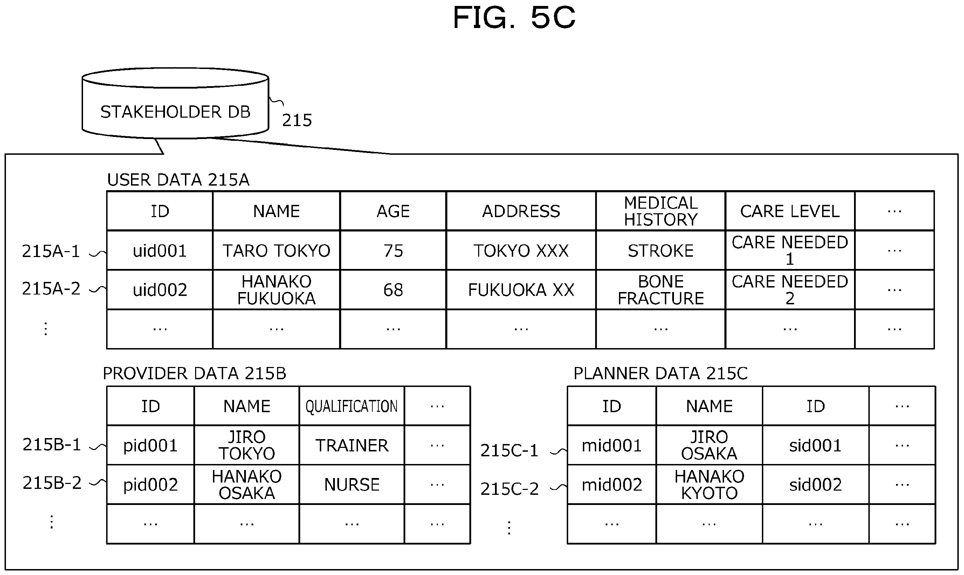

FIG. 5C illustrates the stakeholder DB 215. The stakeholder DB 215 includes user data 215A, provider data 215B, and planner data 215C for each stakeholder.

The user data 215A is stored as a record 215A-a in which information is set in each field on the basis of user information. For example, each field stores information such as a user ID, a name, an age, an address, a medical history, a care level, and the like. For example, the record 215A-1 stores information such as the user ID "uid001", the name "Taro Tokyo", the age "75", the address "Tokyo XXX", the medical history "stroke", the care level "care needed 1", and the like.

The provider data 215B is stored as a record 215B-a in which information is set in each field on the basis of provider information. For example, each field stores information such as a provider ID, a name, a qualification, and the like. For example, the record 215B-1 stores information such as the provider ID "pid001", the name "Jiro Tokyo", the qualification "trainer", and the like.

The planner data 215C is stored as a record 215C-a in which information is set in each field on the basis of planner information. For example, each field stores information such as a planner ID, a name, a menu ID, and the like. For example, the record 215C-1 stores information such as the planner ID "mid001", the name "Jiro Osaka", and the menu ID "sid001".

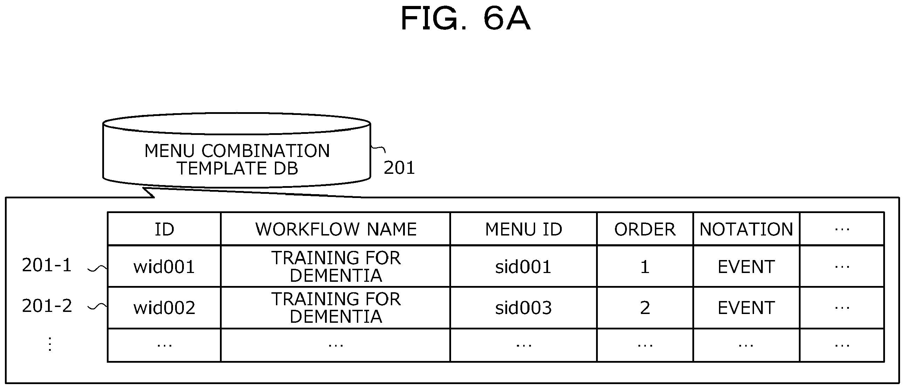

FIGs. 6A to 6D are charts illustrating data examples in DBs retained by the planning device. FIG. 6A illustrates the menu combination template DB 201. The menu combination template DB 201 is stored as a record 201-a in which information is set in each field on the basis of information of a workflow combining rehabilitation menus. For example, each field stores information such as an ID by each workflow, a workflow name, a menu ID, an order, a notation, and the like. For example, the record 201-a stores information such as the workflow ID "wid001", the workflow name "training for dementia", the menu ID "sid001", the order "1", the notation "event", and the like.

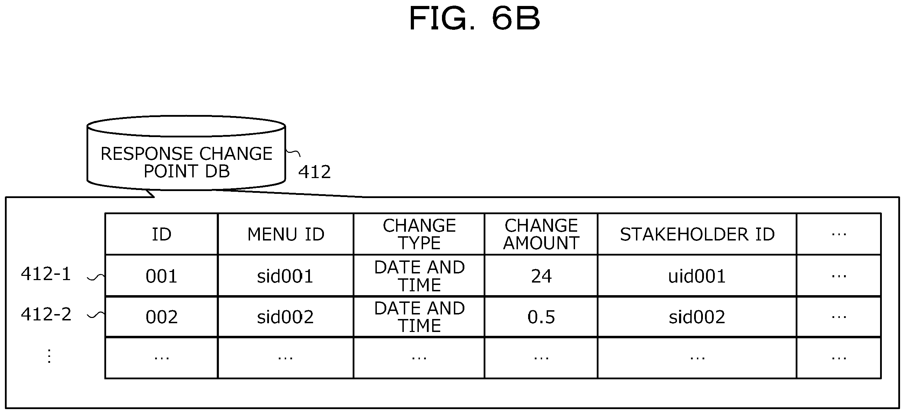

FIG. 6B illustrates the response change point DB 412. The response change point DB 412 is stored as a record 412-a in which information is set in each field on the basis of the information related to the response change for each menu. For example, each field stores information such as an ID, a menu ID, a change type related to the response change point detected by the response change point extraction unit 402, a change amount, a stakeholder ID, and the like. For example, the record 412-1 stores information such as the ID "001", the menu ID "sid001", the change type "date and time", the change amount "24", the stakeholder ID "uid001", and the like.

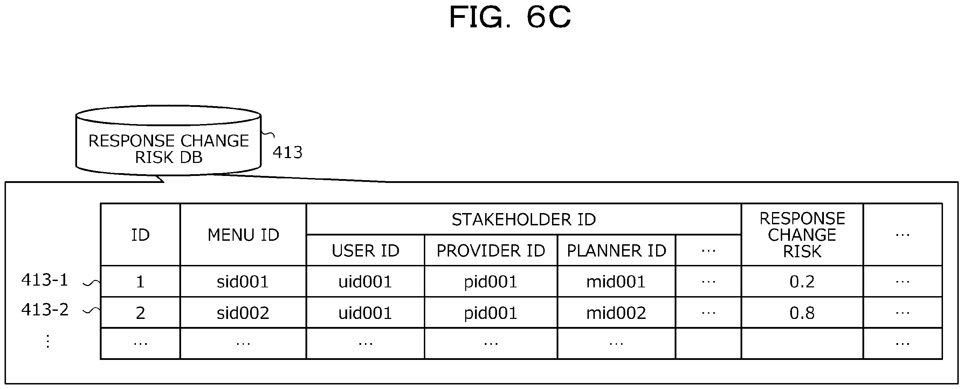

FIG. 6C illustrates the response change risk DB 413. The response change risk DB 413 is stored as a record 413-a in which information is set in each field on the basis of the information of the estimated response change risk. For example, each field stores information such as an ID, a menu ID, an ID of each stakeholder, the response change risk, and the like. For example, the record 413-1 stores information such as the ID "1", the menu ID "sid001", the user ID "uid001", the provider ID "pid001", the planner ID "mid001", a value of the response change risk "0.2", and the like.

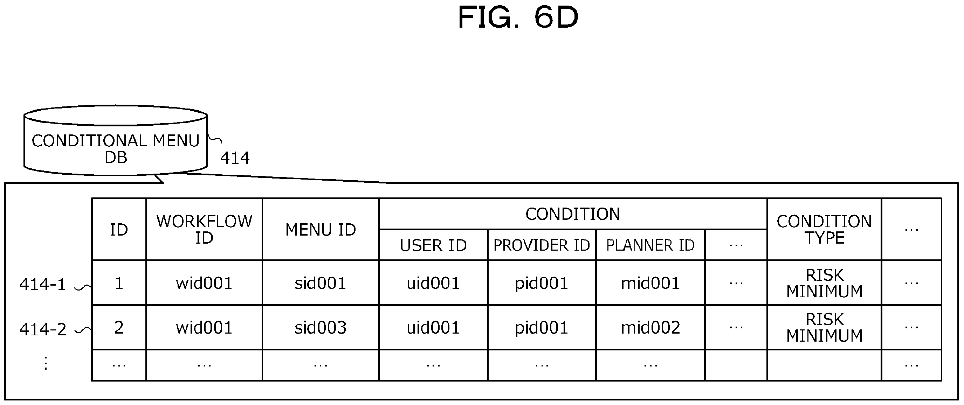

FIG. 6D illustrates the conditional menu DB 414. The conditional menu DB 414 is stored as a record 414-a in which information is set in each field on the basis of the information related to the menu with the extracted condition that minimizes the response change risk. For example, each field stores information such as an ID, a workflow ID, a menu ID, an ID of each stakeholder that satisfies the condition, a condition type, and the like. For example, the record 414-1 stores information such as the ID "1", the workflow ID "wid001", the menu ID "sid001", the user ID "uid001", the provider ID "pid001", the planner ID "mid001", the condition type "risk minimum", and the like.

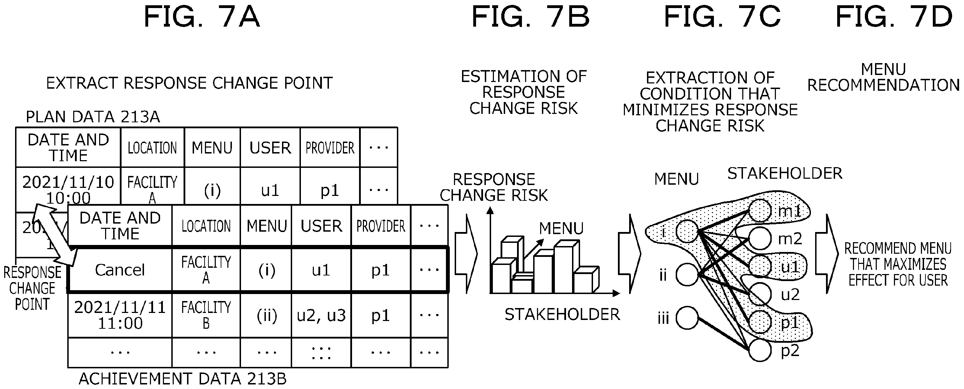

FIG. 7 (i.e., FIGs. 7A to 7D) is an explanatory diagram of an outline of processing performed by the planning device. FIG. 7A illustrates the above-described extraction of the response change point of the process 1. performed by the response change point extraction unit 402. For example, the response change point extraction unit 402 extracts the response change point by acquiring the past plan/achievement DB 213 planned by the planner m1, and comparing the plan data 213A and the achievement data 213B for each record. In the example of FIG. 7A, the response change point extraction unit 402 extracts cancellation of the menu (i) for predetermined date and time planned in the record 213A-1 of the plan data 213A, in the record 213B-1 of the achievement data 213B, as the response change point.

FIG. 7B illustrates the above-described estimation of the response change risk of the process 2. performed by the response change risk estimation unit 403. In FIG. 7B, the horizontal axis is the stakeholder, the vertical axis is the response change risk (change frequency), and the depth axis is the menu. The response change risk estimation unit 403 obtains the response change risk (change frequency) for each stakeholder for a certain menu by extracting the response change points illustrated in FIG. 7A.

FIG. 7C illustrates the above-described extraction of the condition that minimizes the response change risk of the process 3. performed by the risk minimum condition extraction unit 404. The risk minimum condition extraction unit 404 extracts, for a certain menu, a combination of service menus that minimizes the response change risk. In FIG. 7C, the thicker the line between the stakeholders, the higher the response change risk. For example, in a case of providing the menu i to the user u1, the risk minimum condition extraction unit 404 determines that the combination of the planner m1 and the provider p1 has a high response change risk (change frequency) for the menu i. The risk minimum condition extraction unit 404 extracts the combination of stakeholders with a thin line between the stakeholders.

FIG. 7D illustrates the recommendation of the menu that maximizes the effect for the user under the condition extracted in the above-described process 4. performed by the menu recommendation unit 405. The menu recommendation unit 405 recommends, to the user, one or a plurality of menus capable of maximizing the effect for the user among a plurality of menus on the basis of the combination between the stakeholders with the thick line between the stakeholders, which has been extracted by the risk minimum condition extraction unit 404 in FIG. 7C. Furthermore, the menu recommendation unit 405 may also present a schedule for implementing each menu.

Moreover, the menu recommendation unit 405 may recommend, to the user u1, the response change risk for each menu as a constraint condition. For example, the menu recommendation unit 405 may present the information of the conditional menu DB 414 illustrated in FIG. 6D to the user u1, and recommend the menus in a selectable state, in a state of causing the user u1 to recognize the response change risk for each menu.

In the above description, the response change risk estimated by the response change risk estimation unit 403 can be represented by the following expression (1), for example, and can be data-processed by the planning device 100.

$$Pi(C = 1|u, p, m) = \{Pi(u|C, p, m) \times Pi(C|p, m)\}/Pi(u|p, m) \ ... \ (1)$$

The above expression (1) indicates a probability that the menu i planned by the certain planner m and scheduled to be provided by the certain provider p to the certain user u will be changed (C = 1). $Pi(u|C, p, m)$ can be estimated from a statistic for each user u in a case where the certain provider p provides the menu i on the plan of the certain planner m, and a result is C. $Pi(C|p, m)$ can be estimated from a change/non-change statistic in the case where the certain provider p provides the menu i on the plan of the certain planner m. $Pi(u|p, m)$ can be estimated from a statistic for each user u in the case where the certain provider p provides the menu i on the plan of the certain planner m.

Then, in the case of recommending the menu that minimizes the response change risk, the menu recommendation unit 405 selects the provider p and the planner m that maximize $Pi(C = 0, u|p, m)$. The menu recommendation unit 405 recommends, to the user u, "the menu i by the provider p planned by the planner m" with the lowest response change risk.

According to the above description, the planning device 100 of the embodiment performs the processing of expanding a search space by adding attribute values of the response change risks of the plurality of stakeholders to the target menu before outputting the menu to the user u. For example, the existing technique corresponds to plotting and searching for a menu on a search space with two axes of effect and number of times (number of times of use), and does not take into account how (when, who, and where) the menu is provided. In contrast, the planning device 100 of the embodiment corresponds to increasing the axes on the space, using the three-axis search space including the response change risk, the effect, and the number of times. Therefore, according to the planning device 100 of the embodiment, how the menu is be provided can be considered as the response change risk.

(Details of Processing by Each Function of Planning Device 100)

Next, a processing example by each function of the planning device 100 will be described in detail.

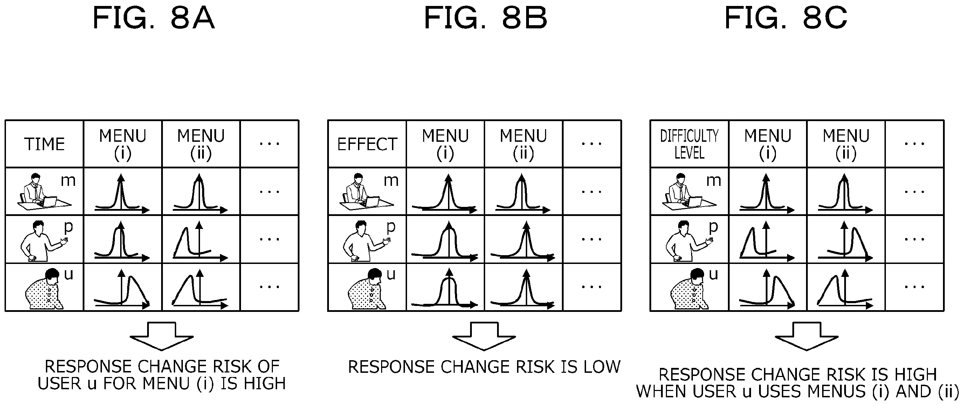

FIGs. 8A to 8C are explanatory diagrams of processing of estimating the response change risk. A processing example of the above-described estimation of the response change risk of the process 2. performed by the planning device 100 will be described. The planning device 100 estimates the response change risk on the basis of a tendency that the response change (change frequency) is more likely to occur in a case where each stakeholder's expected value for service implementation is different. The expected value for service implementation is various types of information (parameters are time, location, effect, difficulty level, and the like) regarding service implementation of each stakeholder. The response change risk increases in a case where the range of expected values differs from that of other stakeholders.

An example in which the response change risk increases will be described.

Example 1: the provider p considered that the menu (i) is moderate training (medium difficulty level). Meanwhile, the user u found it difficult (high difficulty level) after actually receiving the training, and frequently canceled the menu (i) or changed the menu to a similar menu (ii).

Example 2: the planner m thought that the menu (ii) should be implemented in the morning (early hours). Meanwhile, the provider p's work schedule was limited to the afternoon (late hours).

Example 3: the user u wanted to receive the menu (i) on time. Meanwhile, the provider p, who was in charge of the user u, often delayed the start of the menu (i) because it took time to prepare the menu (i).

The planning device 100 refers to the past plan/achievement DB 213, calculates the expected value of each parameter for service implementation for each stakeholder at the extracted response change point, and estimates the response change risk from an overlap of the ranges (distributions).

Regarding each parameter is, for example, the time is the time of service implementation or the like. The location is a location, a distance, or the like of the service implementation. The effect is a service implementation time, a measured value, or the like. The difficulty level is a difference between a standard value and the measured value, a provider's skill set, a user's profile, or the like.

FIG. 8A is a chart illustrating each stakeholder's expected value for each menu in the case where the parameter is a time. The respective expected values (vertical axis) of the planner m, the provider p, and the user u for the menus (i) and (ii) are illustrated. The horizontal axis is a time (for example, time). Each of the planner m, the provider p, and the user u each has a distribution characteristic based on a predetermined time width and height of the expected value. In the example of FIG. 8A, the planning device 100 estimates that the response change risk of the user u to the menu (i) is high.

FIG. 8B is a chart illustrating each stakeholder's expected value for each menu in the case where the parameter is effect. The respective expected values (vertical axis) of the planner m, the provider p, and the user u for the menus (i) and (ii) are illustrated. The horizontal axis is a time (for example, a service implementation time). Each of the planner m, the provider p, and the user u each has a distribution characteristic based on a predetermined time width and height of the expected value. In the example of FIG. 8B, the planning device 100 estimates that each stakeholder's response change risk for the menus (i) and (ii) is low.

FIG. 8C is a chart illustrating each stakeholder's expected value for each menu in the case where the parameter is the difficulty level. The respective expected values (vertical axis) of the planner m, the provider p, and the user u for the menus (i) and (ii) are illustrated. The horizontal axis is a time (for example, a service implementation time). Each of the planner m, the provider p, and the user u each has a distribution characteristic based on a predetermined time width and height of the expected value. In the example of FIG. 8C, the planning device 100 estimates that the response change risk is high when the user u uses the menus (i) and (ii) provided by the provider p.

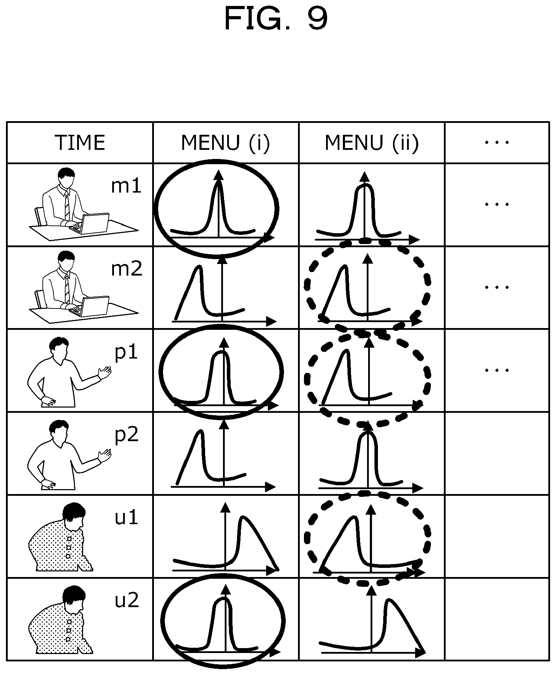

FIG. 9 is an explanatory diagram of extraction processing of conditions that minimize the response change risks. A processing example of the above-described extraction of the condition that minimizes the response change risk of the process 3. performed by the planning device 100 will be described. In the embodiment, attention is focused on the fact that the response change is less likely to occur for a combination of the stakeholders (planners, providers, or users) having the same expectation range regarding service implementation. The planning device 100 groups the stakeholders on the basis of the estimated response change risk, in other words, the similarity of distributions of expectations regarding service implementation, and recommends a menu for each target group.

FIG. 9 is a chart illustrating each stakeholder's expected value for each menu. The respective expected values (vertical axis) of the planner m, the provider p, and the user u for the menus (i) and (ii) are illustrated. The horizontal axis represents a time. In the example of FIG. 9, the planning device 100 determines that the distributions of expectations of the planner m1, the provider pi, and the user u2 are similar for the menu i (the solid line frames in FIG. 9). Furthermore, the planning device 100 determines that the distributions of expectations of the planner m2, the provider pi, and the user u1 are similar for the menu ii (the dotted line frames in FIG. 9).

Furthermore, the planning device 100 utilizes the response change risk and the expected value for scheduling. For example, attention is focused on the fact that the change at the time of menu implementation can be suppressed when the response change risk of the provider p for the menu is low. For example, the planning device 100 sets the menu to be assigned and the response change risk of the provider p as constraint conditions when scheduling work for the provider p, and assigns the menu that minimizes the response change risk. The menu and the response change risk (an example of constraint condition) of the provider p are indicated by the following expression (2).

[Math. 1]

$$Pi\ (C=0,p) = \sum_{u,m} Pi\ (C=0,p \mid u,m) \cdots (2)$$

The expression (2) above indicates the probability that the provider p will provide the menu i without changing the menu (C = 0). The planning device 100 performs the assignment of the menu i to the provider p such that this probability becomes higher.

Furthermore, attention is focused on the fact that the user u's expected value for the menu i can be used for user u's preference for the menu i. When scheduling the provision of the menu i for the user u, the planning device 100 sets the time (date and time) of the assigned menu and the range of expected values (upper limit, lower limit, middle, and the like) for the location as constraint conditions. For example, it is determined that the response change of the user is less likely to occur in a case where the certain user u has a schedule close to a median of the expected value for the time in the certain user u. Meanwhile, in a case where there is another user u with the same expected value, the assignment can be adjusted to a schedule close to the upper limit or lower limit.

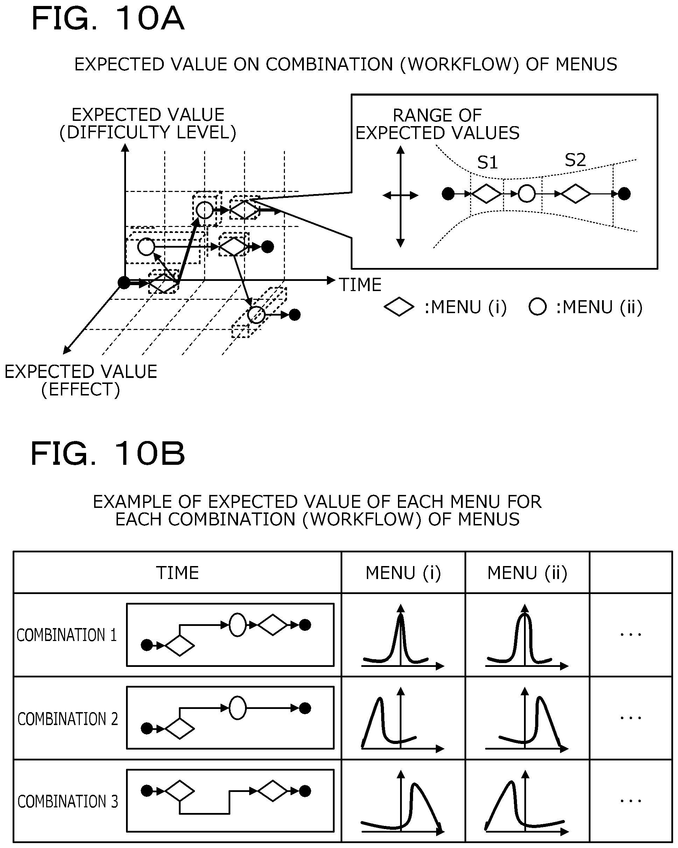

FIGs. 10A and 10B are explanatory diagrams of expected value calculation processing in consideration of a workflow. The planning device 100 calculates the expected value considering a workflow (a combination of menus) when recommending a menu. Depending on the menu, various types of information (time, location, effect, and difficulty level) in service implementation may be determined according to menus implemented before and after the menu. For example, since the preceding menu is a menu that measures the number of times, an end time is ambiguous, but the subsequent menu is a group implementation menu, so the location and start time are strict, or the like. The planning device 100 extracts combinations of menus from the plan and calculates the expected value of each menu for each combination of menus in consideration of such a case.

For example, when estimating the response change risk, the planning device 100 extracts templates of the combinations of menus from the plan data in advance, and calculates the expected value for each menu for each template. Then, the planning device 100 utilizes the expected value for grouping when extracting the condition that minimizes the response change risk. The grouping includes a combination of the menu, the user, the provider, and the planner.

FIG. 10A illustrates the expected values on the combination (workflow) of menus. In FIG. 10A, the horizontal axis represents the time, the vertical axis represents the expected value (for example, the difficulty level), and the axis in a front direction represents the effect. Furthermore, a rhombus (◊) represents the menu i, a circle (○) represents the menu ii, and a rectangular frame represents the range of expected values. The plurality of menus in the workflow can be combined as indicated by the arrows between a start point (●) and an end point (●). At the time of this combination, the planning device 100 combines the menus i and ii on the basis of the ranges of expected values for each of the menus i and ii. For example, in a certain combination, the menu i (S1) has a narrow range of expected values, so the menu needs to be executed strictly in terms of a time, while the menu i (S2) has a wide range of expected values, so the menu can be executed loosely in terms of a time.

FIG. 10B is a chart illustrating the expected value of each menu for each of combinations 1 to 3 (workflows) of menus in the case where the parameter is a time. The respective expected values (vertical axis) of the planner m, the provider p, and the user u for the menus (i) and (ii) are illustrated. The horizontal axis is a time (for example, time). In the example of the combination 1, the expected values for the time for both the menus i and ii are close to the median, so the planning device 100 combines these menus i and ii as a schedule. Furthermore, in the example of the combination 2, the expected value of the menu i deviates from the median to an early side, and the expected value of the menu ii deviates from the median to a late side. The planning device 100 combines these menus i and ii as a schedule. Furthermore, in the example of the combination 3, the expected value of the menu i deviates from the median to a late side, and the expected value of the menu ii deviates from the median to an early side. The planning device 100 combines these menus i and ii as a schedule.

(Processing Example of Planning Device)

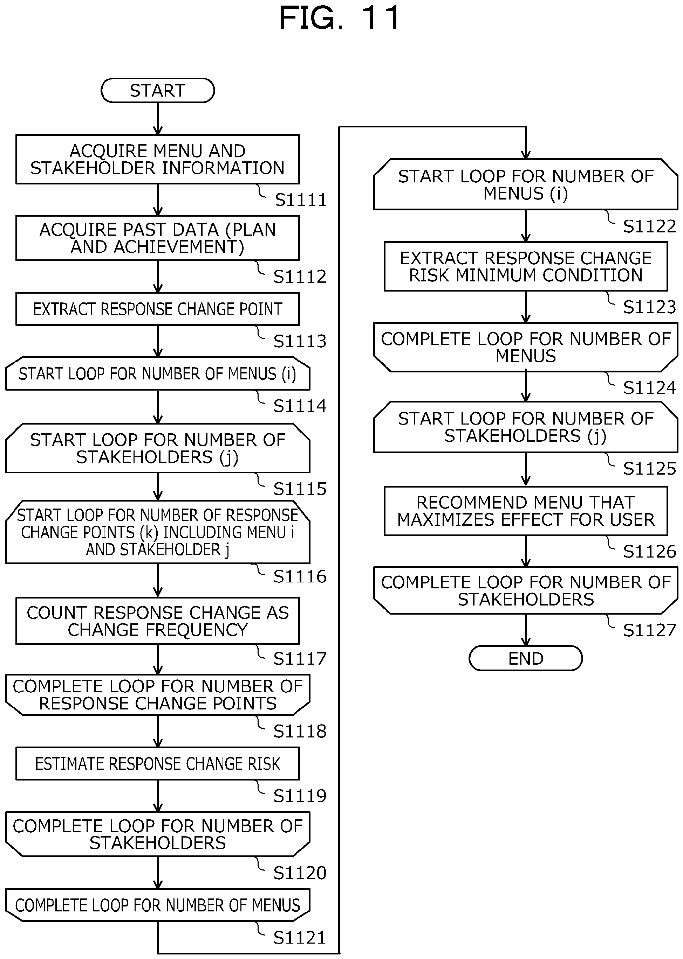

FIG. 11 is a flowchart illustrating an overall processing example of the planning device. A processing example performed by the control unit (CPU 301) of the planning device 100 will be described. The planning device 100 performs the following processing every predetermined cycle, for example. First, the planning device 100 acquires the menus and the stakeholder information (step S1111). Next, the planning device 100 acquires past data (the plan data and the achievement data) (step S1112).

Next, the planning device 100 performs the processing of extracting the response change point (step S1113). Next, the planning device 100 starts loop processing for the number of menus (i) (steps S1114 to S1121). Next, the planning device 100 starts loop processing for the number of stakeholders (j) (steps S1115 to S1120). Next, the planning device 100 starts loop processing for the number of response change points (k) including the menu i and the stakeholder j (steps S1116 to S1118).

Next, the planning device 100 counts the response change as the menu change frequency (step S1117). Next, the planning device 100 determines whether or not the processing regarding the number of response change points k has been completed (step S1118). The planning device 100 returns to step S1116 when the processing for the number of response change points k has not been completed, and proceeds to processing of step S1119 when the processing for the number of response change points k has been completed.

Next, the planning device 100 performs the processing of estimating the response change risk (step S1119). Next, the planning device 100 determines whether or not the processing regarding the number of stakeholders j has been completed (step S1120). The planning device 100 returns to step S1115 when the processing for the number of stakeholders j has not been completed, and proceeds to the processing of step S1121 when the processing for the number of stakeholders j has been completed.

Next, the planning device 100 determines whether or not the processing regarding the number of menus i has been completed (step S1121). The planning device 100 returns to step S1114 when the processing for the number of menus i has not been completed, and proceeds to processing of step S1122 when the processing for the number of menus i has been completed.

Next, the planning device 100 starts loop processing for the number of menus (i) (steps S1122 to S1124). Next, the planning device 100 performs the processing of extracting the response change risk minimum condition (step S1123). Next, the planning device 100 determines whether or not the processing regarding the number of menus i has been completed (step S1124). The planning device 100 returns to step S1122 when the processing for the number of menus i has not been completed, and proceeds to processing of step S1125 when the processing for the number of menus i has been completed.

Next, the planning device 100 starts loop processing for the number of stakeholders (j) (steps S1125 to S1127).

Next, the planning device 100 recommends the menu that maximizes the effect for the user (step S1126). Next, the planning device 100 determines whether or not the processing regarding the number of stakeholders j has been completed (step S1127). The planning device 100 returns to step S1125 when the processing for the number of stakeholders j has not been completed, and terminates the above processing when the processing for the number of stakeholders j has been completed.

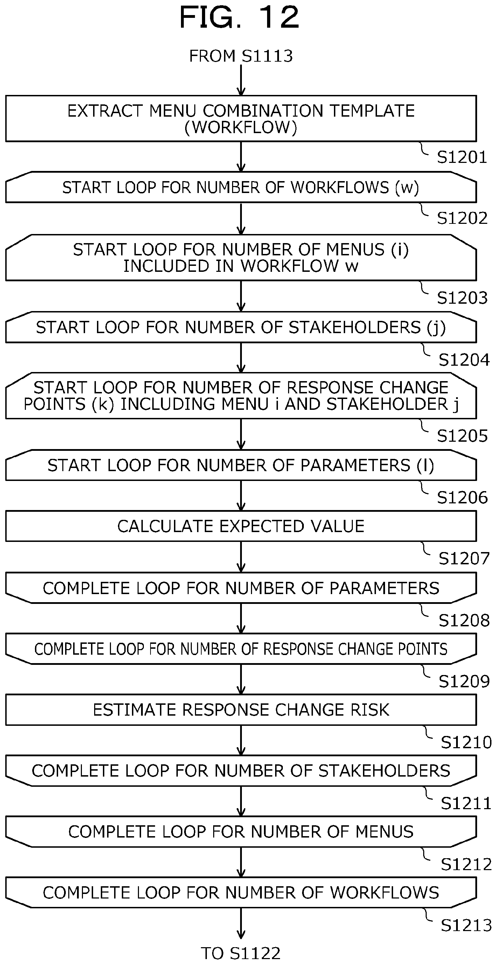

FIG. 12 is a flowchart illustrating a calculation processing example of the expected value and the response change risk in consideration of a workflow. Processing in FIG. 12 is processing obtained by adding calculation processing for the expected value and the response change risk in consideration of a workflow to the processing illustrated in FIG. 11. FIG. 12 corresponds to the processing portion of steps S1114 to S1121 in FIG. 11.

First, the planning device 100 extracts the menu combination template (workflow) (step S1201) after the processing of extracting the response change point (step S1113). Next, the planning device 100 starts loop processing for the number of workflows (w) (steps S1202 to S1213). Next, the planning device 100 starts loop processing for the number of menus (i) included in the workflow w (steps S1203 to S1212). Next, the planning device 100 starts loop processing for the number of stakeholders (j) (steps S1204 to S1211). Next, the planning device 100 starts loop processing for the number of response change points (k) including the menu i and the stakeholder j (steps S1205 to S1209). Next, the planning device 100 starts loop processing for the number of parameters (I) (steps S1206 to S1208).

Next, the planning device 100 calculates the expected value (see FIGs. 10A and 10B, step S1207). Next, the planning device 100 determines whether or not the processing regarding the number of parameters I has been completed (step S1208). The planning device 100 returns to step S1206 when the processing for the number of parameters I has not been completed, and proceeds to processing of step S1209 when the processing for the number of parameters I has been completed.

Next, the planning device 100 determines whether or not the processing regarding the number of response change points k has been completed (step S1209). The planning device 100 returns to step S1205 when the processing for the number of response change points k has not been completed, and proceeds to processing of step S1210 when the processing for the number of response change points k has been completed. In step S1210, the planning device 100 performs the processing of estimating the response change risk (step S1210).

Next, the planning device 100 determines whether or not the processing regarding the number of stakeholders j has been completed (step S1211). The planning device 100 returns to step S1204 when the processing for the number of stakeholders j has not been completed, and proceeds to the processing of step S1212 when the processing for the number of stakeholders j has been completed.

Next, the planning device 100 determines whether or not the processing regarding the number of menus i has been completed (step S1212). The planning device 100 returns to step S1203 when the processing for the number of menus i has not been completed, and proceeds to processing of step S1213 when the processing for the number of menus i has been completed.

Next, the planning device 100 determines whether or not the processing regarding the number of workflows w has been completed (step S1213). The planning device 100 returns to step S1202 when the processing for the number of workflows w has not been completed, and terminates the above processing and proceeds to the processing of step S1122 (see FIG. 11) when the processing for the number of workflows w has been completed.

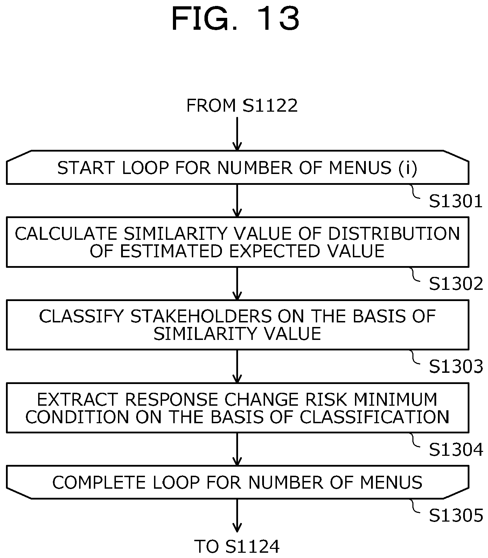

FIG. 13 is a flowchart illustrating a processing example of extraction of a response change risk minimum condition. FIG. 13 illustrates a detailed example of the processing in step S1123 of FIG. 11. First, the planning device 100 starts loop processing for the number of menus (i) (steps S1301 to S1305).

Next, the planning device 100 calculates the similarity of the distributions of the estimated expected values (step S1302). Next, the planning device 100 classifies the stakeholders on the basis of the similarity (step S1303). Next, the planning device 100 extracts the minimum condition for the response change risk on the basis of the classification (step S1304). Next, the planning device 100 determines whether or not the processing regarding the number of menus i has been completed (step S1305). The planning device 100 returns to step S1301 when the processing for the number of menus i has not been completed, and terminates the above processing and proceeds to processing of step S1124 when the processing for the number of menus i has been completed.

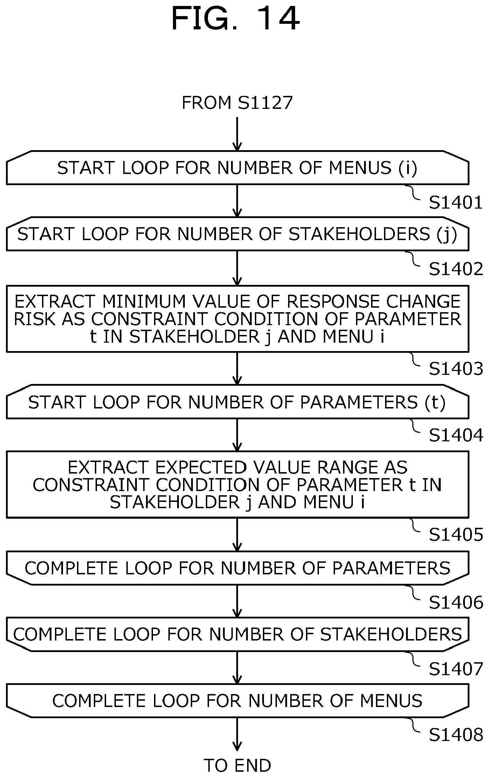

FIG. 14 is a flowchart illustrating a processing example of scheduling of a response change risk and an expected value. The processing illustrated in FIG. 14 is an example of utilizing the response change risk and the expected value for scheduling, and can be executed after the processing of step S1127 in FIG. 11.

The planning device 100 starts loop processing for the number of menus (i) after the processing of step S1127 in FIG. 11 (steps S1401 to S1408). Next, the planning device 100 starts loop processing for the number of stakeholders (j) (steps S1402 to S1407).

Next, the planning device 100 extracts a minimum value of the response change risk as a constraint condition for a parameter t in the stakeholder j and the menu i (step S1403). Next, the planning device 100 starts loop processing for the number of parameters (t) (steps S1404 to S1406).

Next, the planning device 100 extracts the range of expected values as a constraint condition for the parameter t in the stakeholder j and the menu i (step S1405). Next, the planning device 100 returns to step S1404 when the processing for the number of parameters t has not been completed, and proceeds to processing of step S1407 when the processing for the number of parameters t has been completed.

Next, the planning device 100 determines whether or not the processing regarding the number of stakeholders j has been completed (step S1407). The planning device 100 returns to step S1402 when the processing for the number of stakeholders j has not been completed, and proceeds to the processing of step S1408 when the processing for the number of stakeholders j has been completed. Next, the planning device 100 determines whether or not the processing regarding the number of menus i has been completed (step S1408). The planning device 100 returns to step S1401 when the processing for the number of menus i has not been completed, and terminates the above processing (proceeds to END in FIG. 11) when the processing for the number of menus i has been completed.

<Specific Example of Processing by Planning Device>

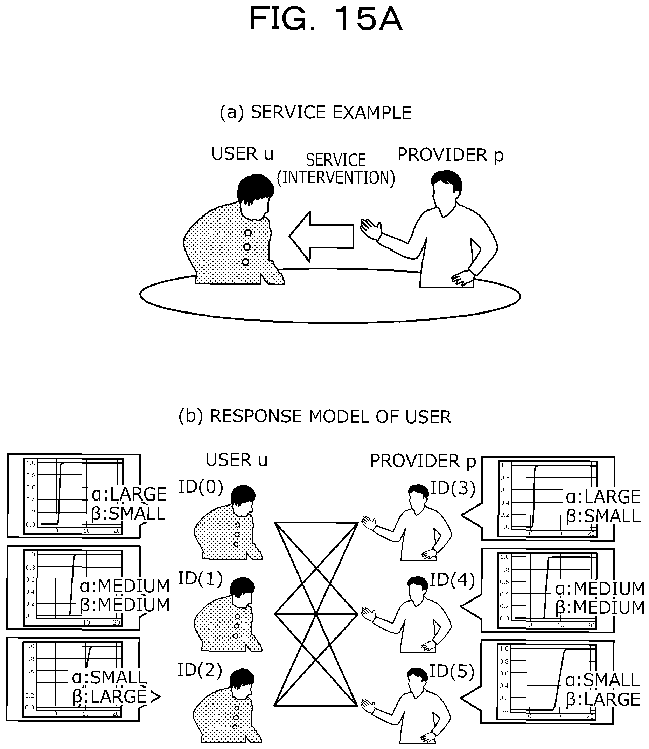

Next, a specific example of the processing by the planning device 100 will be described. FIG. 15A is explanatory diagrams illustrating an example of the user and the provider of a service.

FIG. 15A(a) is a diagram illustrating a service example. For example, in the planning device 100, one provider p provides one user u with a service. In this case, the stakeholders are the user u and the provider p.

FIG. 15A(b) is a diagram illustrating a user's response model. The planning device 100 uses different response models for each user u and the provider p. The planning device 100 randomly determines a combination of the user u and the provider p, and determines the service to be provided according to a response of the user u and the provider p of each determined combination. A plurality of users u (ID = 0 to 2) and providers p (ID = 03 to 5) respectively has respective values of time violation sensitivities $\alpha$ and $\beta$ (large and small notation in FIG. 15A(b)). Details of $\alpha$ and $\beta$ will be described below.

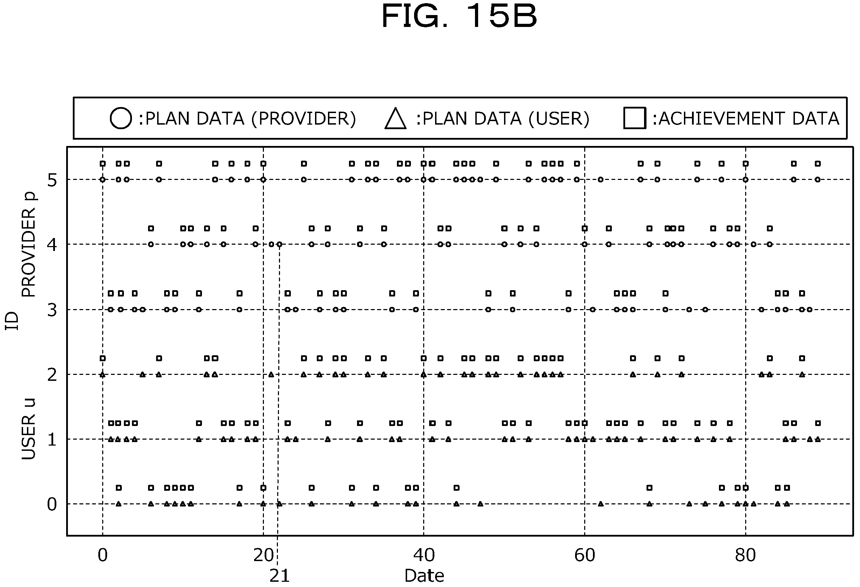

FIG. 15B is a diagram illustrating an example of the plan data and the achievement data. The horizontal axis of FIG. 15B represents a time (date), and the vertical axis represents each user u (ID = 0 to 2) and each provider p (ID = 3 to 5) illustrated in FIG. 15A. In FIG. 15B, ○ represents the provider p's plan data, △ represents the user u's plan data, and □ represents the achievement data. The planning device 100 presents to the user u the date on which the provider p's plan data ○ matches the user u's plan data △. Meanwhile, for the user u, the date on which the service was actually used among the presented dates is indicated by □.

For example, on the date (20), since the plan data ○ of the provider p (ID = 5) matches the plan data △ of the user u (ID = 0), the planning device 100 presents the user u information of service use on this date. Then, the achievement data □ that the user u actually used the service on this date is illustrated.

In contrast, on the date (21), since the plan data ○ of the provider p (ID = 4) matches the plan data △ of the user u (ID = 0), the planning device 100 presents the user u information of service use on this date. However, it is illustrated that the user u did not actually use the service (there is no achievement data □).

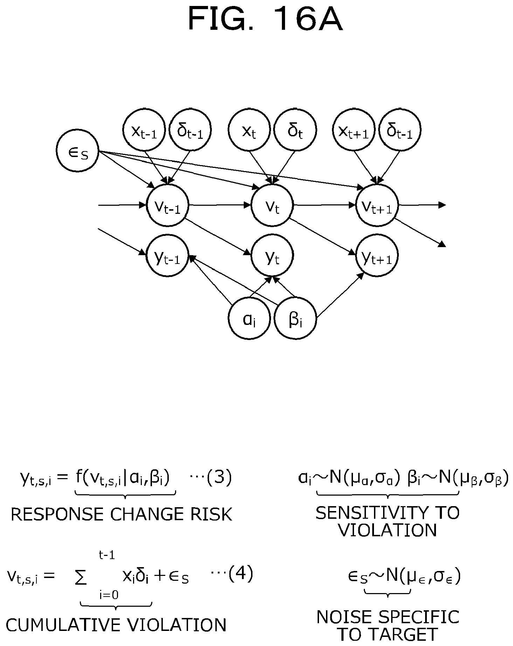

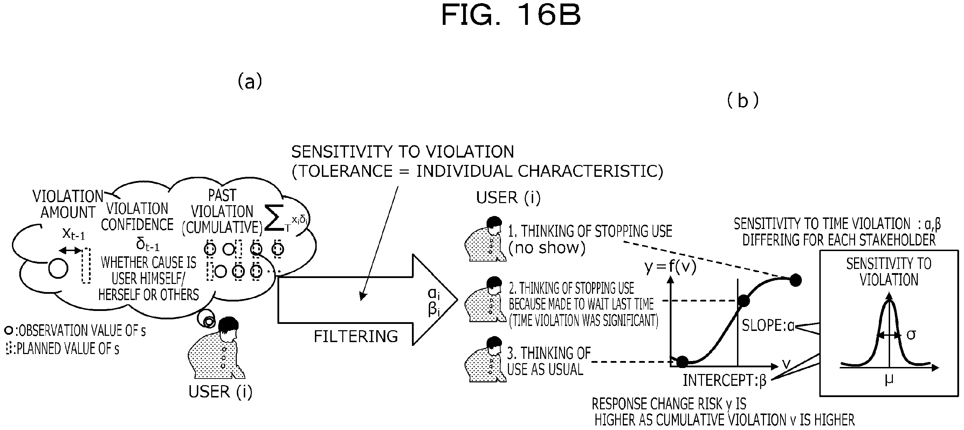

FIGs. 16A and 16B are explanatory diagrams of an estimation example of a response change risk. The planning device 100 estimates the response change risk using the sensitivity to time violation as an expected value (parameter). As illustrated in FIG. 16A, the response change risk of the stakeholder i for a target s at time t is illustrated in the expression (3) for $y_{t,s,i}$. Cumulative violation is illustrated in the expression (4) for $v_{t,s,i}$. $\alpha_i$ and $\beta_i$ are the sensitivities to the violation, and $\varepsilon_s$ is a noise specific to the target s. The horizontal axis of FIG. 16A represents a time, and the figure illustrates a relationship among the noise $\varepsilon_s$ related to the cumulative violation $V_t$, a violation amount $X_t$, a violation confidence value $\delta_t$, the cumulative violation $V_t$ related to the response change $y_t$, and the sensitivities $\alpha_i$ and $\beta_i$.

As illustrated in FIG. 16B(a), in the case where the stakeholder i is the user, regarding expected violation for the target s, the past cumulative violation is given by the above expression (4) where ○ represents an observation value of s, a dotted line rectangle □ represents a planned value of s, the violation amount $X_{t-1}$, and the violation confidence $\delta_{t-1}$. Deviation amounts of ○ with respect to positions of the plurality of rectangles □ in FIG. 16B(a) correspond to past violations, and the planning device 100 cumulates (Σ) the past violations. Then, the planning device 100 filters $\alpha_i$ and $\beta_i$ as expected values (parameters) as the sensitivities (tolerances) to violations, and estimates the response change risk.

Here, as illustrated in FIG. 16B(b), a characteristic curve of a function of the response change y = f(v) is given where the horizontal axis is v and the vertical axis is y. On the characteristic curve, a range where both y and v are large corresponds to 1. the user i is thinking about stopping using the service. A range where both y and v are in a central portion corresponds to 2. the user i is thinking about stopping using the service because the user was made to wait last time (the time violation was significant), for example. A range where both y and v are small corresponds to 3. the user i is thinking about use of the service as usual.

In FIG. 16B(b), the larger the cumulative violation v, the higher the response change risk y. When the curve exceeds the intercept $\beta$, the number of users who stop using the service increases. The slope $\alpha$ and the intercept $\beta$, which are the sensitivities to the time violation differ for each stakeholder. The sensitivities (slope $\alpha$ and intercept $\beta$) to violation have a predetermined distribution characteristic having a width of $\sigma$ with respect to a median $\mu$, where the

horizontal axis is the time violation with respect to a time and the vertical axis is a ratio.

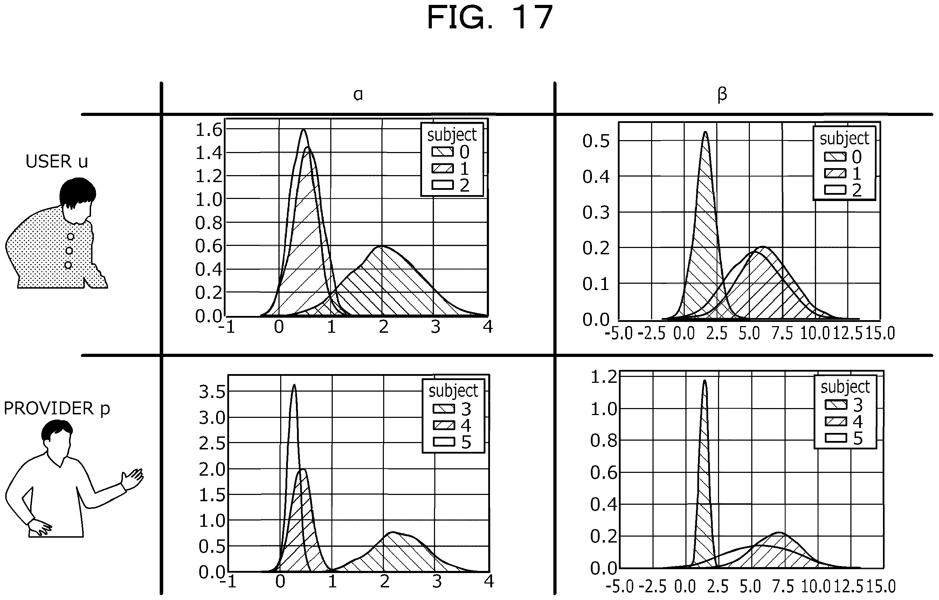

FIG. 17 is a diagram illustrating an estimation example of expected values for each stakeholder. An estimation example of the expected values (parameters) $\alpha$ and $\beta$ for the user u (ID = 0 to 2) and the provider p (ID = 3 to 5) is illustrated. The distribution characteristics of the expected values (parameters) $\alpha$ and $\beta$ differ between the user u (ID = 0 to 2) and the provider p (ID = 3 to 5).

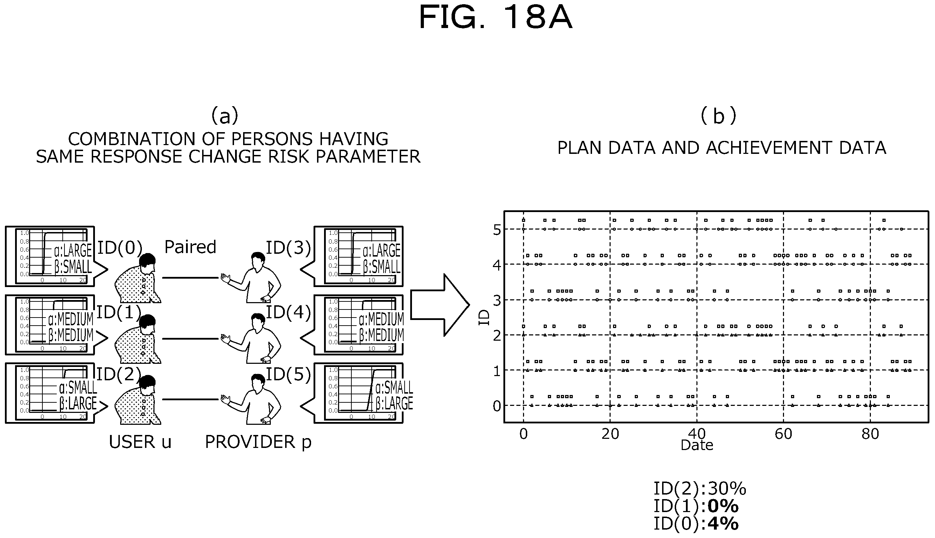

FIG. 18A is an explanatory diagram of a combination example that minimizes a response change risk. FIG. 18A(a) illustrates a state in which persons (stakeholders) who have the same response change risk parameter are combined with each other. The planning device 100 of the embodiment combines the user u (ID = 0) with the provider p (ID = 3) both having $\alpha$: large and $\beta$: small. Furthermore, the planning device 100 combines the user u (ID = 1) with the provider p (ID = 4) both having $\alpha$: medium and $\beta$: medium. Furthermore, the planning device 100 combines the user u (ID = 2) with the provider p (ID = 5) both having $\alpha$: small and $\beta$: large.

FIG. 18A(b) is the plan data and the achievement data at the time of the combinations illustrated in FIG. 18A(a). In the combinations, the rate of plan change is 0% for the user u (ID = 1) and 4% for the user u (ID = 0), indicating that the deviation (response change) of the achievement data with respect to the plan data can be suppressed.

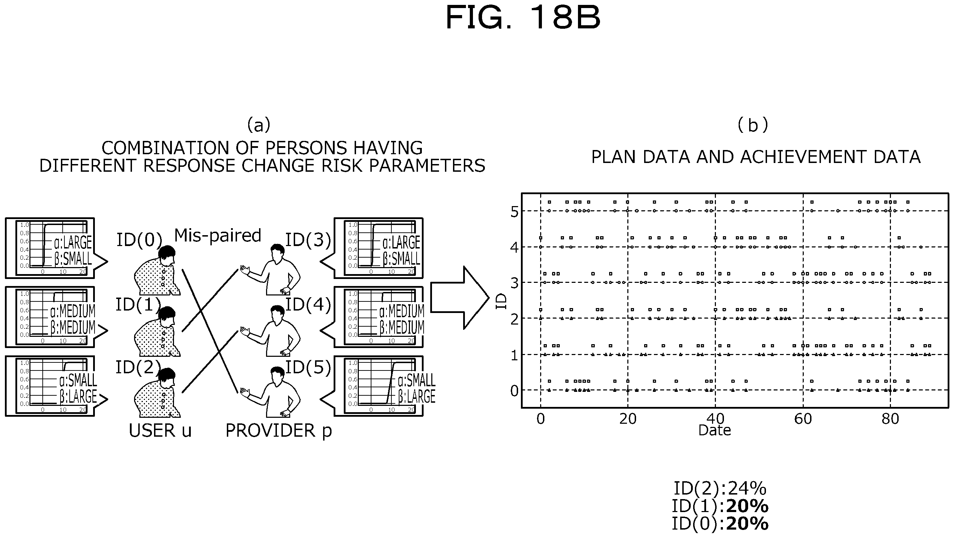

FIG. 18B is an explanatory diagram of a combination example that cannot minimize the response change risk. FIG. 18B is an explanatory diagram for comparison with FIG. 18A. For example, as illustrated in FIG. 18B(a), it is assumed that persons with different response change risk parameters are combined with each other. For example, it is assumed that the user u (ID = 0) having $\alpha$: large and $\beta$: small is combined with the provider p (ID = 5) having $\alpha$: small and $\beta$: large. Furthermore, it is assumed that the user u (ID = 1) having $\alpha$: medium and $\beta$: medium is combined with the provider p (ID = 3) having $\alpha$: large and $\beta$: small. Furthermore, it is assumed that the user u (ID = 2) having $\alpha$: small and $\beta$: large is combined with the provider p (ID = 4) having $\alpha$: medium and $\beta$: medium.

FIG. 18B(b) is the plan data and the achievement data at the time of the combinations illustrated in FIG. 18B(a). In the combinations, the rate of plan change was 20% for the user u (ID = 1) and 20% for the user u (ID = 0). In this way, in the case where persons having different response change risk parameters are combined with each other, the deviation (response change) of the achievement data with respect to the plan data occurs, and the response change risk cannot be minimized.

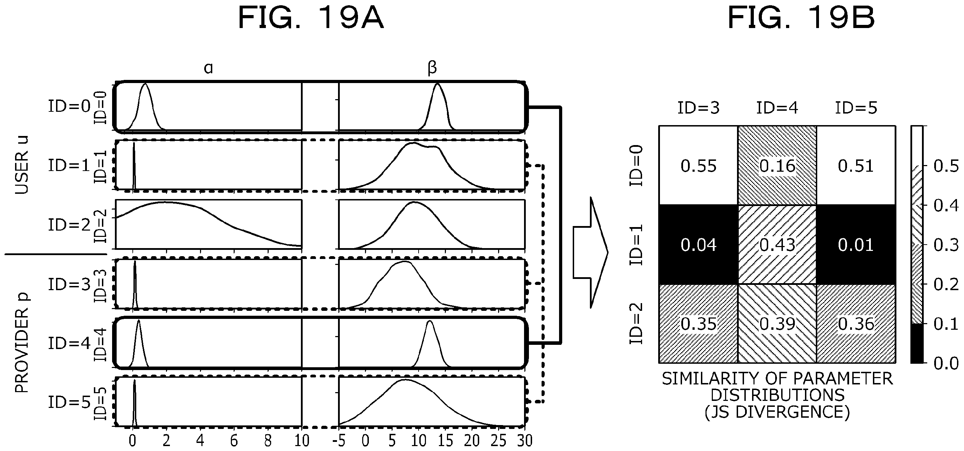

FIG. 19 (FIGs. 19A and 19B) is an explanatory diagram of similarity of estimated expected values. FIG. 19A illustrates $\alpha$ and $\beta$ of each user u and provider p. In FIG. 19B, each user u (ID = 0 to 2) is arranged on the vertical axis and each provider p (ID = 3 to 5) is arranged on the horizontal axis, and the similarity (distribution deviation) between mutual parameter distributions is illustrated. The similarity can be calculated on the basis of, for example, KL divergence or JS divergence.

As illustrated in FIG. 19B, for example, the planning device 100 determines that the provider p (ID = 3) has a value of "0.04" and the provider p (ID = 5) has a value of "0.01", and each of the providers p is similar to the user u (ID = 1). Furthermore, the planning device 100 determines that the provider p (ID = 4) has a value of "0.16" and is similar to the user u (ID = 0).

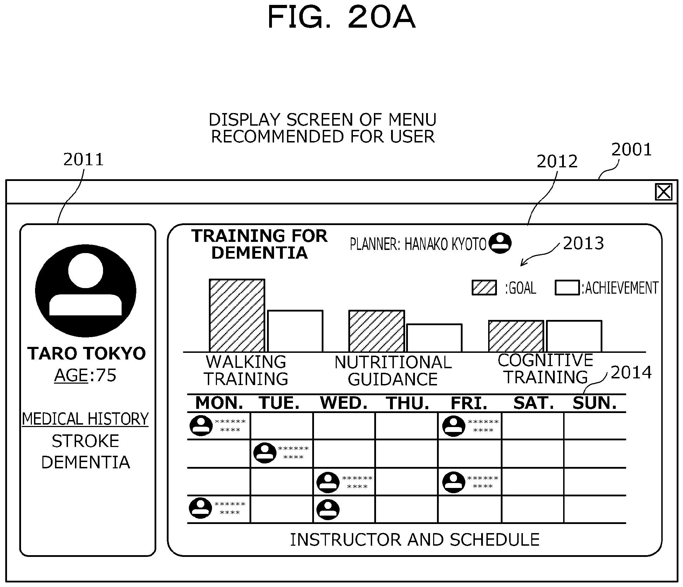

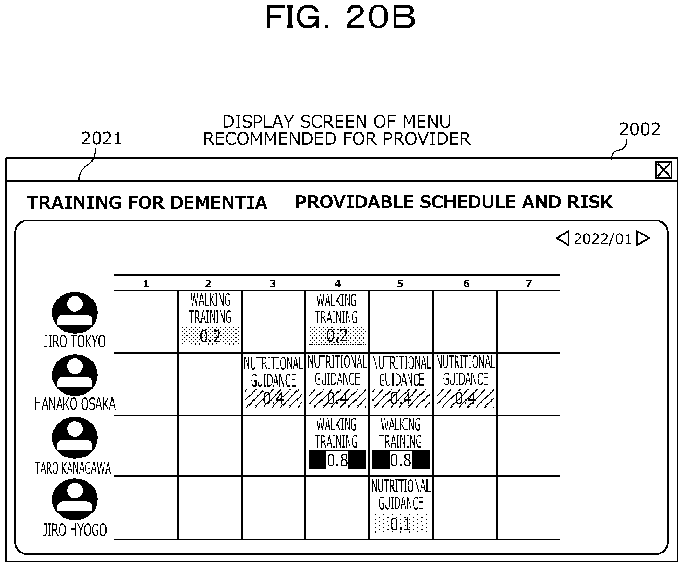

FIGs. 20A and 20B are diagrams illustrating display examples of a recommended menu. The planning device 100 generates screen data, and transmits and outputs the screen data to the client 211 of the user or the provider.

FIG. 20A illustrates a display screen 2001 of the menu recommended to the user. For example, the planning device 100 displays and outputs the combination that minimizes the response change risk for the menu. The planning device 100 displays various types of information of the user (name "Taro Tokyo", age, medical history, and the like) in a left-side user column 2011 of the display screen 2001. The planning device 100 displays information of the menu planned as training for dementia for the user "Taro Tokyo" in a center and right-side menu column 2012 of the display screen 2001. In the menu column 2012, the planning device 100 displays the name of the planner, a graph 2013 of goal and achievement of each menu (walking training, nutritional guidance, cognitive training, or the like), and a schedule table 2014 illustrating an instructor and schedule of each menu for each day of the week.

FIG. 20B illustrates a display screen 2002 of the menu recommended to the provider. For example, the planning device 100 displays and outputs, to the provider, a combination of menus with the response change risk as a constraint condition. The planning device 100 displays each user on the vertical axis and displays a menu schedule table 2021 for each user on the date (January 2022) on the horizontal axis, on the display screen 2002. The planning device 100 displays a menu in a square of the user and the date of the schedule table 2021 and also displays a value of the response change risk (see FIG. 6C). Furthermore, the planning device 100 displays that the risk is high in menu implementation for the corresponding square (the user and the date) by displaying, for example, the corresponding square with a darker color or the like as the value of the response change risk is higher. The provider can perform scheduling in consideration of the risk for each menu recommended by the planning device 100 displayed in the schedule table 2021.

The above-described embodiment has been described using an example in which the planning device 100 recommends a rehabilitation menu for the user as a service. The embodiment is not limited to this example, and the planning device 100 can be similarly applied to various technical training and exercise menus, medical examination and treatment menus at hospitals, various service menus for administration, and the like.

As described above, according to the planning device 100, processing of acquiring information of a menu of a predetermined service and information of a stakeholder related to the service; calculating a change frequency of execution of the menu for a combination of the stakeholder and the menu; specifying a menu to be recommended on the basis of the calculated change frequency; and outputting the specified menu to the stakeholder of the calculated combination is performed. Therefore, the planning device 100 can recommend the menu that is highly likely to be implemented to the stakeholder.

Furthermore, according to the planning device 100, the processing of acquiring performs processing of acquiring plan data that plans menu execution of the service and achievement data obtained by actually executing the menu of the plan data, the processing of calculating performs processing of extracting a change point of presence or absence of the menu execution on the basis of a difference between the acquired plan data and the acquired achievement data, and calculating the change frequency for each of the stakeholders for the menu having the change point, and the processing of specifying performs processing of specifying a combination of the stakeholders with the low change frequency to the menu. Therefore, the planning device 100 can recommend the menu that is more likely to be implemented to the stakeholder on the basis of the past plan data and achievement data.

Furthermore, according to the planning device 100, the processing of calculating the change frequency performs processing of calculating on the basis of an overlap of distributions of parameters including a time, a location, an effect, and a difficulty level that indicate expected values regarding implementation of the menu for each stakeholder. In this way, the planning device 100 can recommend the optimum menu among a plurality of stakeholders, taking into consideration the tendency that the change frequency is more likely to occur in the case where the expected value for service implementation for each stakeholder is different.

Furthermore, according to the planning device 100, the processing of specifying performs processing of combining the stakeholders each other having similar distributions of the expected values. Therefore, the planning device 100 can recommend the optimum menu for each of the combined stakeholders.

Furthermore, according to the planning device 100, the processing of specifying performs processing of obtaining a workflow that combines a plurality of menus on the basis of the distributions of the expected values for each plurality of menus. Therefore, the planning device 100 can recommend a service flow that combines menus that are highly likely to be implemented, for a plurality of menus that constitute a service.

Furthermore, the stakeholders include a planner who plans the menu of the service, a provider who provides the service, and a user who uses the service, and according to the planning device 100, the processing of specifying may include processing of combining the provider and the user with similar distributions of the expected values. Therefore, the planning device 100 can recommend a menu that is highly likely to be implemented to each of the provider and the user.

Furthermore, according to the planning device 100, the processing of specifying performs processing of assigning the menu with the low change frequency using the change frequency of the provider for the menu as a constraint condition, in a case where the change frequency of the provider for the menu is low. Therefore, the planning device 100 can be used for scheduling combining service menus, and feasibility of scheduling by the provider can be improved.

Furthermore, according to the planning device 100, the processing of specifying performs processing of assigning the menu with the distribution of the expected value of the user for the menu as a constraint condition. Therefore, the planning device 100 can improve the feasibility of a plurality of menus by the user.

Furthermore, according to the planning device 100, the processing of outputting performs processing of displaying and outputting the combination of the menus with the low change frequency to the user. As a result, it is possible to specifically recommend a highly feasible menu combination to the user of the service who uses the planning device 100.

Furthermore, according to the planning device 100, the processing of outputting performs processing of displaying and outputting, to the provider, a schedule in which the menus are combined for each user with the change frequency as a constraint condition. Therefore, it is possible to specifically recommend, to the service provider who uses the planning device 100, a highly feasible menu combination to each user according to circumstances of service provision.

Furthermore, according to the planning device 100, the processing of displaying and outputting may include information of the change frequency for each menu. Therefore, the planning device 100 can specifically present the feasibility of each menu of the recommended schedule to the provider, and the provider can create the schedule while grasping the feasibility of the menu.

Note that the planning method described in the present embodiment may be implemented by executing a program prepared in advance on a computer such as a personal computer (PC) or a workstation. The planning program described in the present embodiment is executed by being recorded on a computer-readable recording medium and being read from the recording medium by the computer. The recording medium is a hard disk, a flexible disk, a compact disc (CD)-ROM, a magneto optical disc (MO), a digital versatile disc (DVD), or the like. Furthermore, the planning program described in the present embodiment may be distributed via a network such as the Internet.

**Claims**

1. A computer-implemented method of performing planning processing, the method comprising:

   acquiring information of a menu of a predetermined service and information of a stakeholder related to the service;
   calculating a change frequency of execution of the menu for a combination of the stakeholder and the menu;
   specifying a menu to be recommended on the basis of the calculated change frequency; and
   outputting the specified menu to the stakeholder of the calculated combination.

2. The computer-implemented method according to claim 1, wherein

   the processing of acquiring includes
   acquiring plan data that plans menu execution of the service and achievement data obtained by actually executing the menu of the plan data,
   the processing of calculating includes

   extracting a change point of presence or absence of the menu execution on the basis of a difference between the acquired plan data and the acquired achievement data, and
   calculating the change frequency for each of the stakeholders for the menu that has the change point, and

   the processing of specifying includes
   specifying a combination of the stakeholders with the low change frequency to the menu.

3. The computer-implemented method according to claim 2, wherein
   the processing of calculating the change frequency
   is calculated on the basis of an overlap of distributions of parameters that include a time, a location, an effect, and a difficulty level that indicate expected values regarding implementation of the menu for each of the stakeholders.

4. The computer-implemented method according to claim 3, wherein
   the processing of specifying includes
   combining the stakeholders each other that have similar distributions of the expected values.

5. The computer-implemented method according to claim 3 or 4, wherein
   the processing of specifying includes
   obtaining a workflow that combines a plurality of menus on the basis of the distributions of the expected values for each plurality of menus.

6. The computer-implemented method according to claim 4, wherein

   the stakeholders include a planner who plans the menu of the service, a provider who provides the service, and a user who uses the service, and
   the processing of specifying includes
   processing of combining the provider and the user with similar distributions of the expected values.

7. The computer-implemented method according to claim 6, wherein
   the processing of specifying includes
   assigning the menu with the low change frequency that uses the change frequency of the provider for the menu as a constraint condition, in a case where the change frequency of the provider for the menu is low.

8. The computer-implemented method according to claim 6, wherein
   the processing of specifying includes
   assigning the menu with the distribution of the expected value of the user for the menu as a constraint condition.

9. The computer-implemented method according to claim 8, wherein
   the processing of outputting includes
   displaying and outputting the combination of the menus with the low change frequency to the user.

10. The computer-implemented method according to claim 7, wherein

the processing of outputting includes
displaying and outputting, to the provider, a schedule in which the menus are combined for the each user with the change frequency as a constraint condition.

11. The computer-implemented method according to claim 10, wherein
the processing of displaying and outputting includes
information of the change frequency for the each menu.

12. A planning program comprising instructions which, when executed by a computer, cause the computer to execute processing, the processing comprising:

acquiring information of a menu of a predetermined service and information of a stakeholder related to the service;
calculating a change frequency of execution of the menu for a combination of the stakeholder and the menu;
specifying a menu to be recommended on the basis of the calculated change frequency; and
outputting the specified menu to the stakeholder of the calculated combination.

# FIG. 1

~100

(a) WHICH MENU IS TO BE RECOMMENDED TO USER? SERVICE MENU 1

(b) EXTRACT RESPONSE CHANGE RISK FOR PLURALITY OF COMBINATIONS IN SERVICE MENU IMPLEMENTATION

(c) RECOMMEND MENU WITH COMBINATION OF LOW RESPONSE CHANGE RISK

USER u1

SERVICE MENU 1
SERVICE MENU 2
SERVICE MENU 3

USER u1

PROVIDER p1

PROVIDER p2

PLANNER m1
SERVICE MENU 1
SERVICE MENU 2

CHANGE FREQUENCY
PROVIDER p3
PROVIDER p1
PROVIDER p2
p1 p2 p3 ...

PLANNER m2
SERVICE MENU 3
SERVICE MENU 4

CHANGE FREQUENCY
p1 p2 p3 ...
PROVIDER p1   PROVIDER p2   PROVIDER p3

USER u1

SERVICE MENU 1

SERVICE MENU 2   PLANNER m1   PROVIDER p1

SERVICE MENU 3   PLANNER m2   PROVIDER p2

EP 4 220 657 A1

# FIG. 2

PLANNING DEVICE

201

MENU COMBINATION
TEMPLATE DB

200

SERVER

100

NW

210

REHABILITATION
FACILITY A

212 — HUB

CLIENT

211

PAST PLAN/
ACHIEVEMENT DB — 213

MENU DB — 214

STAKEHOLDER DB — 215

210

REHABILITATION
FACILITY B

212 — HUB

CLIENT

211

PAST PLAN/
ACHIEVEMENT DB — 213

MENU DB — 214

STAKEHOLDER DB — 215

. . .

EP 4 220 657 A1

# FIG. 3

EP 4 220 657 A1

# FIG. 4

PAST PLAN/ ACHIEVEMENT DB — 213

MENU DB — 214

STAKEHOLDER DB — 215

210

SERVER — 200

MENU COMBINATION TEMPLATE DB — 201

401 PAST DATA ACQUISITION UNIT

402 RESPONSE CHANGE POINT EXTRACTION UNIT

403 RESPONSE CHANGE RISK ESTIMATION UNIT

404 RISK MINIMUM CONDITION EXTRACTION UNIT

405 MENU RECOMMENDATION UNIT

RESPONSE CHANGE POINT DB — 412

RESPONSE CHANGE RISK DB — 413

CONDITIONAL MENU DB — 414

EP 4 220 657 A1

# FIG. 5A

PAST PLAN/ACHIEVEMENT DB  213

PLAN DATA 213A

| | ID | DATE AND TIME | FACILITY ID | MENU ID | USER ID | PROVIDER ID | ⋯ |
|---|---|---|---|---|---|---|---|
| 213A-1 | 001 | 2021/11/10 10:00 | fid001 | sid001 | uid001 | pid001 | ⋯ |
| 213A-2 | 002 | 2021/11/11 11:00 | fid002 | sid002 | uid002 | pid001 | ⋯ |
| ⋮ | ⋯ | ⋯ | ⋯ | ⋯ | ⋯ | ⋯ | ⋯ |

ACHIEVEMENT DATA 213B

| | ID | DATE AND TIME | FACILITY ID | MENU ID | USER ID | PROVIDER ID | ⋯ |
|---|---|---|---|---|---|---|---|
| 213B-1 | 001 | Cancel | - | - | - | - | ⋯ |
| 213B-2 | 002 | 2021/11/11 11:30 | fid002 | sid002 | uid002 | pid001 | ⋯ |
| ⋮ | ⋯ | ⋯ | ⋯ | ⋯ | ⋯ | ⋯ | ⋯ |

EP 4 220 657 A1

# FIG. 5B

MENU DB 〜 214

| ID | MENU NAME | TIME REQUIRED | STANDARD VALUE | UNIT | ... |
|---|---|---|---|---|---|
| sid001 | WALKING TRAINING | 30 MINUTES | 10 | STEP | ... |
| sid002 | NUTRITIONAL GUIDANCE | 60 MINUTES | 60 | MINUTE | ... |
| ... | ... | ... | ... | ... | ... |

214-1

214-2

# FIG. 5C

STAKEHOLDER DB 215

USER DATA 215A

| ID | NAME | AGE | ADDRESS | MEDICAL HISTORY | CARE LEVEL | ... |
|---|---|---|---|---|---|---|
| uid001 | TARO TOKYO | 75 | TOKYO XXX | STROKE | CARE NEEDED 1 | ... |
| uid002 | HANAKO FUKUOKA | 68 | FUKUOKA XX | BONE FRACTURE | CARE NEEDED 2 | ... |
| ... | ... | ... | ... | ... | ... | ... |

215A-1, 215A-2

PROVIDER DATA 215B

| ID | NAME | QUALIFICATION | ... |
|---|---|---|---|
| pid001 | JIRO TOKYO | TRAINER | ... |
| pid002 | HANAKO OSAKA | NURSE | ... |
| ... | ... | ... | ... |

215B-1, 215B-2

PLANNER DATA 215C

| ID | NAME | ID | ... |
|---|---|---|---|
| mid001 | JIRO OSAKA | sid001 | ... |
| mid002 | HANAKO KYOTO | sid002 | ... |
| ... | ... | ... | ... |

215C-1, 215C-2

EP 4 220 657 A1

# FIG. 6A

MENU COMBINATION TEMPLATE DB — 201

| | ID | WORKFLOW NAME | MENU ID | ORDER | NOTATION | … |
|---|---|---|---|---|---|---|
| 201-1 | wid001 | TRAINING FOR DEMENTIA | sid001 | 1 | EVENT | … |
| 201-2 | wid002 | TRAINING FOR DEMENTIA | sid003 | 2 | EVENT | … |
| ⋮ | … | … | … | … | … | … |

# FIG. 6B

RESPONSE CHANGE POINT DB — 412

| ID | MENU ID | CHANGE TYPE | CHANGE AMOUNT | STAKEHOLDER ID | ... |
|---|---|---|---|---|---|
| 001 | sid001 | DATE AND TIME | 24 | uid001 | ... |
| 002 | sid002 | DATE AND TIME | 0.5 | sid002 | ... |
| ... | ... | ... | ... | ... | ... |

412-1

412-2

FIG. 6C

EP 4 220 657 A1

RESPONSE CHANGE RISK DB — 413

| ID | MENU ID | STAKEHOLDER ID | | | | RESPONSE CHANGE RISK | ... |
| | | USER ID | PROVIDER ID | PLANNER ID | ... | | |
|----|---------|---------|-------------|------------|-----|------|-----|
| 1 | sid001 | uid001 | pid001 | mid001 | ... | 0.2 | ... |
| 2 | sid002 | uid001 | pid001 | mid002 | ... | 0.8 | ... |
| ... | ... | ... | ... | ... | | ... | ... |

413-1

413-2

# FIG. 6D

EP 4 220 657 A1

CONDITIONAL MENU DB — 414

| ID | WORKFLOW ID | MENU ID | CONDITION | | | | CONDITION TYPE | ... |
|----|-------------|---------|-----------|----|----|----|----------------|-----|
|    |             |         | USER ID | PROVIDER ID | PLANNER ID | ... |                |     |
| 1  | wid001      | sid001  | uid001  | pid001      | mid001     | ... | RISK MINIMUM   | ... |
| 2  | wid001      | sid003  | uid001  | pid001      | mid002     | ... | RISK MINIMUM   | ... |
| ... | ...        | ...     | ...     | ...         | ...        |     |                | ... |

414-1

414-2

## FIG. 7A

EXTRACT RESPONSE CHANGE POINT

PLAN DATA 213A

| DATE AND TIME | LOCATION | MENU | USER | PROVIDER | ··· |
|---|---|---|---|---|---|
| 2021/11/10 10:00 | FACILITY A | (i) | u1 | p1 | ··· |

| DATE AND TIME | LOCATION | MENU | USER | PROVIDER | ··· |
|---|---|---|---|---|---|
| Cancel | FACILITY A | (i) | u1 | p1 | ··· |
| 2021/11/11 11:00 | FACILITY B | (ii) | u2, u3 | p1 | ··· |
| ··· | ··· | ··· | ⋮ | ··· | ··· |

RESPONSE CHANGE POINT

ACHIEVEMENT DATA 213B

## FIG. 7B

ESTIMATION OF RESPONSE CHANGE RISK

RESPONSE CHANGE RISK

MENU

STAKEHOLDER

## FIG. 7C

EXTRACTION OF CONDITION THAT MINIMIZES RESPONSE CHANGE RISK

MENU    STAKEHOLDER

i    m1
     m2
ii   u1
     u2
iii  p1
     p2

## FIG. 7D

MENU RECOMMENDATION

RECOMMEND MENU THAT MAXIMIZES EFFECT FOR USER

## FIG. 8A

| TIME | MENU (i) | MENU (ii) | ... |
|---|---|---|---|
| m | | | ... |
| p | | | ... |
| u | | | ... |

RESPONSE CHANGE RISK OF
USER u FOR MENU (i) IS HIGH

## FIG. 8B

| EFFECT | MENU (i) | MENU (ii) | ... |
|---|---|---|---|
| m | | | ... |
| p | | | ... |
| u | | | ... |

RESPONSE CHANGE RISK IS LOW

## FIG. 8C

| DIFFICULTY LEVEL | MENU (i) | MENU (ii) | ... |
|---|---|---|---|
| m | | | ... |
| p | | | ... |
| u | | | ... |

RESPONSE CHANGE RISK IS HIGH
WHEN USER u USES MENUS (i) AND (ii)

EP 4 220 657 A1

# FIG. 9

# FIG. 10A

## EXPECTED VALUE ON COMBINATION (WORKFLOW) OF MENUS

# FIG. 10B

## EXAMPLE OF EXPECTED VALUE OF EACH MENU FOR EACH COMBINATION (WORKFLOW) OF MENUS

# FIG. 11

START

ACQUIRE MENU AND STAKEHOLDER INFORMATION — S1111

ACQUIRE PAST DATA (PLAN AND ACHIEVEMENT) — S1112

EXTRACT RESPONSE CHANGE POINT — S1113

START LOOP FOR NUMBER OF MENUS (i) — S1114

START LOOP FOR NUMBER OF STAKEHOLDERS (j) — S1115

START LOOP FOR NUMBER OF RESPONSE CHANGE POINTS (k) INCLUDING MENU i AND STAKEHOLDER j — S1116

COUNT RESPONSE CHANGE AS CHANGE FREQUENCY — S1117

COMPLETE LOOP FOR NUMBER OF RESPONSE CHANGE POINTS — S1118

ESTIMATE RESPONSE CHANGE RISK — S1119

COMPLETE LOOP FOR NUMBER OF STAKEHOLDERS — S1120

COMPLETE LOOP FOR NUMBER OF MENUS — S1121

START LOOP FOR NUMBER OF MENUS (i) — S1122

EXTRACT RESPONSE CHANGE RISK MINIMUM CONDITION — S1123

COMPLETE LOOP FOR NUMBER OF MENUS — S1124

START LOOP FOR NUMBER OF STAKEHOLDERS (j) — S1125

RECOMMEND MENU THAT MAXIMIZES EFFECT FOR USER — S1126

COMPLETE LOOP FOR NUMBER OF STAKEHOLDERS — S1127

END

# FIG. 12

FROM S1113

EXTRACT MENU COMBINATION TEMPLATE (WORKFLOW) S1201

START LOOP FOR NUMBER OF WORKFLOWS (w) S1202

START LOOP FOR NUMBER OF MENUS (i) INCLUDED IN WORKFLOW w S1203

START LOOP FOR NUMBER OF STAKEHOLDERS (j) S1204

START LOOP FOR NUMBER OF RESPONSE CHANGE POINTS (k) INCLUDING MENU i AND STAKEHOLDER j S1205

START LOOP FOR NUMBER OF PARAMETERS (l) S1206

CALCULATE EXPECTED VALUE S1207

COMPLETE LOOP FOR NUMBER OF PARAMETERS S1208

COMPLETE LOOP FOR NUMBER OF RESPONSE CHANGE POINTS S1209

ESTIMATE RESPONSE CHANGE RISK S1210

COMPLETE LOOP FOR NUMBER OF STAKEHOLDERS S1211

COMPLETE LOOP FOR NUMBER OF MENUS S1212

COMPLETE LOOP FOR NUMBER OF WORKFLOWS S1213

TO S1122

# FIG. 13

FROM S1122

START LOOP FOR NUMBER OF MENUS (i)
S1301

CALCULATE SIMILARITY VALUE OF DISTRIBUTION
OF ESTIMATED EXPECTED VALUE
S1302

CLASSIFY STAKEHOLDERS ON THE BASIS OF
SIMILARITY VALUE
S1303

EXTRACT RESPONSE CHANGE RISK MINIMUM
CONDITION ON THE BASIS OF CLASSIFICATION
S1304

COMPLETE LOOP FOR NUMBER OF MENUS
S1305

TO S1124

# FIG. 14

FROM S1127

START LOOP FOR NUMBER OF MENUS (i)
S1401

START LOOP FOR NUMBER OF STAKEHOLDERS (j)
S1402

EXTRACT MINIMUM VALUE OF RESPONSE CHANGE RISK AS CONSTRAINT CONDITION OF PARAMETER t IN STAKEHOLDER j AND MENU i
S1403

START LOOP FOR NUMBER OF PARAMETERS (t)
S1404

EXTRACT EXPECTED VALUE RANGE AS CONSTRAINT CONDITION OF PARAMETER t IN STAKEHOLDER j AND MENU i
S1405

COMPLETE LOOP FOR NUMBER OF PARAMETERS
S1406

COMPLETE LOOP FOR NUMBER OF STAKEHOLDERS
S1407

COMPLETE LOOP FOR NUMBER OF MENUS
S1408

TO END

# FIG. 15A

## (a) SERVICE EXAMPLE

USER u    SERVICE    PROVIDER p
(INTERVENTION)

## (b) RESPONSE MODEL OF USER

USER u        PROVIDER p

α:LARGE    ID(0)        ID(3)    α:LARGE
β:SMALL                         β:SMALL

α:MEDIUM   ID(1)        ID(4)    α:MEDIUM
β:MEDIUM                        β:MEDIUM

α:SMALL    ID(2)        ID(5)    α:SMALL
β:LARGE                         β:LARGE

# FIG. 15B

Legend: ○ :PLAN DATA (PROVIDER)   △ :PLAN DATA (USER)   □ :ACHIEVEMENT DATA

Y-axis: ID — PROVIDER p (5, 4, 3), USER u (2, 1, 0)
X-axis: Date (0, 20, 21, 40, 60, 80)

# FIG. 16A

$$y_{t,s,i} = \underbrace{f(v_{t,s,i} | a_i, \beta_i)} \quad \cdots (3)$$

RESPONSE CHANGE RISK

$$v_{t,s,i} = \underbrace{\sum_{i=0}^{t-1} x_i \delta_i} + \epsilon_s \quad \cdots (4)$$

CUMULATIVE VIOLATION

$$a_i \sim \underbrace{N(\mu_a, \sigma_a) \; \beta_i \sim N(\mu_\beta, \sigma_\beta)}$$

SENSITIVITY TO
VIOLATION

$$\epsilon_s \sim \underbrace{N(\mu_\epsilon, \sigma_\epsilon)}$$

NOISE SPECIFIC
TO TARGET

# FIG. 16B

(a)

(b)

SENSITIVITY TO VIOLATION
(TOLERANCE = INDIVIDUAL CHARACTERISTIC)

VIOLATION AMOUNT — VIOLATION CONFIDENCE — PAST VIOLATION (CUMULATIVE) $\sum_T x_i \delta_i$

$x_{t-1}$

$\delta_{t-1}$

WHETHER CAUSE IS USER HIMSELF/HERSELF OR OTHERS

O :OBSERVATION VALUE OF s
:PLANNED VALUE OF s

USER (i)

FILTERING

$\alpha_i$
$\beta_i$

USER (i)

1. THINKING OF STOPPING USE (no show)

2. THINKING OF STOPPING USE BECAUSE MADE TO WAIT LAST TIME (TIME VIOLATION WAS SIGNIFICANT)

3. THINKING OF USE AS USUAL

SENSITIVITY TO TIME VIOLATION : $\alpha, \beta$
DIFFERING FOR EACH STAKEHOLDER

$y = f(v)$

SLOPE: $\alpha$

INTERCEPT: $\beta$

RESPONSE CHANGE RISK y IS
HIGHER AS CUMULATIVE VIOLATION v IS HIGHER

SENSITIVITY TO VIOLATION

$\sigma$

$\mu$

EP 4 220 657 A1

# FIG. 17

# FIG. 18A

## (a)
### COMBINATION OF PERSONS HAVING
### SAME RESPONSE CHANGE RISK PARAMETER

## (b)
### PLAN DATA AND ACHIEVEMENT DATA

ID(0) Paired ID(3)

α:LARGE
β:SMALL

ID(1)

α:MEDIUM
β:MEDIUM

ID(4)

ID(2)

α:SMALL
β:LARGE

ID(5)

α:LARGE
β:SMALL

α:MEDIUM
β:MEDIUM

α:SMALL
β:LARGE

USER u    PROVIDER p

ID(2):30%
ID(1):**0%**
ID(0):**4%**

# FIG. 18B

(a)
COMBINATION OF PERSONS HAVING
DIFFERENT RESPONSE CHANGE RISK PARAMETERS

(b)
PLAN DATA AND ACHIEVEMENT DATA

ID(2):24%
ID(1):**20%**
ID(0):**20%**

EP 4 220 657 A1

# FIG. 19A

# FIG. 19B

SIMILARITY OF PARAMETER
DISTRIBUTIONS
(JS DIVERGENCE)

# FIG. 20A

DISPLAY SCREEN OF MENU
RECOMMENDED FOR USER

2011     2012   2001

**TARO TOKYO**
AGE:75

MEDICAL HISTORY
STROKE
DEMENTIA

**TRAINING FOR DEMENTIA**   PLANNER: HANAKO KYOTO   2013

▨ :GOAL    ☐ :ACHIEVEMENT

WALKING TRAINING    NUTRITIONAL GUIDANCE    COGNITIVE TRAINING   2014

| MON. | TUE. | WED. | THU. | FRI. | SAT. | SUN. |
|------|------|------|------|------|------|------|
| ☻ ****** **** | | | | ☻ ****** **** | | |
| | ☻ ****** **** | | | | | |
| | | ☻ ****** **** | | ☻ ****** **** | | |
| ☻ ****** **** | | ☻ | | | | |

INSTRUCTOR AND SCHEDULE

# FIG. 20B

DISPLAY SCREEN OF MENU
RECOMMENDED FOR PROVIDER

2021                                                              2002

**TRAINING FOR DEMENTIA    PROVIDABLE SCHEDULE AND RISK**

◁ 2022/01 ▷

| | 1 | 2 | 3 | 4 | 5 | 6 | 7 |
|---|---|---|---|---|---|---|---|
| JIRO TOKYO | | WALKING TRAINING 0.2 | | WALKING TRAINING 0.2 | | | |
| HANAKO OSAKA | | | NUTRITIONAL GUIDANCE 0.4 | NUTRITIONAL GUIDANCE 0.4 | NUTRITIONAL GUIDANCE 0.4 | NUTRITIONAL GUIDANCE 0.4 | |
| TARO KANAGAWA | | | | WALKING TRAINING ■0.8■ | WALKING TRAINING ■0.8■ | | |
| JIRO HYOGO | | | | | NUTRITIONAL GUIDANCE 0.1 | | |

Europäisches
Patentamt

European
Patent Office

Office européen
des brevets

# EUROPEAN SEARCH REPORT

**Application Number**

EP 22 20 6609

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/302358 A1 (MCBRIDE MATTHEW [US] ET AL) 24 September 2020 (2020-09-24) * paragraph [0002] - paragraph [0021] * ----- | 1-12 | INV. G16H40/00 G06Q10/0631 |
| X | US 2010/106517 A1 (KOCIUBINSKI MARIUSZ [US] ET AL) 29 April 2010 (2010-04-29) * paragraph [0009] - paragraph [0011] * * paragraph [0016] - paragraph [0019]; figure 2 * * paragraph [0022] - paragraph [0025] * ----- | 1-12 | |
| X | US 2009/089092 A1 (JOHNSON CHRISTOPHER [US] ET AL) 2 April 2009 (2009-04-02) * paragraph [0030] - paragraph [0053]; figure 4 * ----- | 1-12 | |

**TECHNICAL FIELDS SEARCHED (IPC)**

G16H
G06Q

The present search report has been drawn up for all claims

| Place of search | Date of completion of the search | Examiner |
|---|---|---|
| Munich | 17 May 2023 | Eichenauer, Lars |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 6609

This annex lists the patent family members relating to the patent documents cited in the above-mentioned European search report.
The members are as contained in the European Patent Office EDP file on
The European Patent Office is in no way liable for these particulars which are merely given for the purpose of information.

17-05-2023

| Patent document cited in search report | | Publication date | Patent family member(s) | | Publication date |
|---|---|---|---|---|---|
| US 2020302358 | A1 | 24-09-2020 | NONE | | |
| US 2010106517 | A1 | 29-04-2010 | NONE | | |
| US 2009089092 | A1 | 02-04-2009 | US | 2009089092 A1 | 02-04-2009 |
| | | | US | 2009089093 A1 | 02-04-2009 |
| | | | US | 2009112618 A1 | 30-04-2009 |
| | | | US | 2012010901 A1 | 12-01-2012 |
| | | | US | 2014303996 A1 | 09-10-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020166835 A
- JP 2021509505 A
- JP 2008165358 A

- WO 2018030340 A
- US 10923233 B
- US 20170300637

**Non-patent literature cited in the description**

- **TOMOMI OGAWA ; HIROKI MATSUMOTO.** Improvement of Rehabilitation Menu Recommendation System with Extended Nursing Care Need Level Determination Period. *Journal of Telemedicine Society of Japan,* 2020, vol. 16 (2), 152-155

- It is possible to propose an optimal rehabilitation plan that matchesthe characteristics of each user of nursing care services ~ Verifi cation of the eff ectiveness of individual optimal technologyusing digital data in nursing rehabilitation ~. NTT DATA INSTITUTE OF MANAGEMENT CONSULTING, INC, 01 July 2021